# EUROPÄISCHE PATENTSCHRIFT

(11) **EP 3 942 096 B1**
(45) Veröffentlichungstag und Bekanntmachung des Hinweises auf die Patenterteilung: **06.08.2025**
(21) Anmeldenummer: 21716196.7
(22) Anmeldetag: 01.04.2021
(51) Int. Cl.: C25F 3/06, C25F 3/24, C23C 22/50, C23G 1/00, C23G 1/08, B24C 1/10, A61B 17/00

(54) **VERFAHREN ZUM OBERFLÄCHENBEHANDELN EINES CHIRURGISCHEN INSTRUMENTS, SOWIE CHIRURGISCHES INSTRUMENT**
METHOD FOR SURFACE TREATMENT OF A SURGICAL INSTRUMENT, AND SURGICAL INSTRUMENT
PROCÉDÉ DE TRAITEMENT DE SURFACE D'UN INSTRUMENT CHIRURGICAL, ET INSTRUMENT CHIRURGICAL

(30) Priorität: 06.04.2020 DE 102020204431
(43) Veröffentlichungstag der Anmeldung: 26.01.2022
(73) Patentinhaber: Aesculap AG, 78532 Tuttlingen (DE)
(72) Erfinder: GASSNER, Andreas, 78532 Tuttlingen (DE); WAIDELICH, Lukas, 78532 Tuttlingen (DE)
(74) Vertreter: Patentanwälte Ruff, Wilhelm, Beier, Dauster & Partner mbB
(86) Internationale Anmeldenummer: PCT/EP2021/058723
(87) Internationale Veröffentlichungsnummer: WO 2021/204702

(56) Entgegenhaltungen:
- FR-A1- 2 149 542
- RU-C1- 2 046 158
- SE-C1- 143 429
- US-A1- 2017 114 472
- STÖVER M ET AL: "Microstructuring of stainless steel implants by electrochemical etching", JOURNAL OF MATERIAL SCIENCE, KLUWER ACADEMIC PUBLISHERS, DORDRECHT, vol. 41, no. 17, 1 September 2006 (2006-09-01), pages 5569 - 5575, XP036654088, ISSN: 0022-2461, [retrieved on 20060901], DOI: 10.1007/S10853-006-0257-7
- ANONYMUS: "Technologies for Marking Surgical Instruments Guidance Document", 1 January 2017 (2017-01-01), pages 1 - 8, XP055815653, Retrieved from the Internet <URL:https://www.gs1uk.org/sites/default/files/gs1_uk_hug_technologies_marking_surgical_instruments_docV2.pdf> [retrieved on 20210618]

## Beschreibung

### ANWENDUNGSGEBIET UND STAND DER TECHNIK

Die Erfindung betrifft ein Verfahren zum Herstellen eines chirurgischen Instruments oder eines Bauteils davon, sowie ein chirurgisches Instrument oder ein Bauteil davon.

Medizintechnische Produkte, wie insbesondere chirurgische Instrumente, werden vor ihrer Fertigstellung in der Regel einer Oberflächenbehandlung unterworfen. Hierzu können die Oberflächen der Produkte beispielsweise mittels Gleit- und/oder Bandschleifen bearbeitet werden. Dadurch können Fehler im Vormaterial und/oder schmiedebedingte Fehler, wie beispielsweise entkohlte Bereiche, oder Oberflächenfehler, wie beispielsweise Poren, Narben oder Risse, beseitigt werden, welche sich anderenfalls nachteilig auf die Korrosionsbeständigkeit der Produkte auswirken würden.

Allerdings können durch Bandschleifen feine Kerben oder Anhebungen auf der Produktoberfläche entstehen. Diese können bei einem nachfolgenden Behandlungsschritt umgebogen oder eingedrückt werden. Dadurch können Materialdopplungen entstehen. Außerdem kann es zu einem vereinzelten Materialübertrag, beispielsweise von Siliziumoxidpartikeln, von einem Schleifband auf die Produktoberfläche kommen. Ein derartiger Materialübertrag und die mit der mechanischen Bearbeitung einhergehende Belastung des medizintechnischen Produkts können wiederum Eigenspannungen im Produkt erzeugen oder erhöhen. Problematisch ist zudem, dass beim Schleifen nicht beseitigte oder erzeugte Oberflächenfehler des Produkts in einem nachfolgenden Behandlungsschritt nur noch begrenzt beseitigt werden können.

Zur Mattierung eines medizintechnischen Produkts können sphärische Strahlmittel, wie beispielsweise Glasperlen, verwendet werden. Dadurch kommt es zu einer plastischen Verformung der Produktoberfläche, wodurch diese vergrößert und aufgeraut wird. Da Glasperlen im Allgemeinen sehr hart (Härte 6 Mohs) und obendrein spröde sind, kommt es im Laufe der Zeit zu einem teilweisen Bruch des Strahlmittels. Dadurch treffen während des Mattierungsschrittes sowohl sphärische Glasperlen als auch gebrochene Glasperlen auf die Oberfläche des Produkts. Während gebrochene Glasperlen scharfe Kerben auf der Produktoberfläche erzeugen, hinterlassen ungebrochene Glasperlen sphärische Eindrücke auf der Oberfläche des Produkts. Durch das Auftreffen von gebrochenen und ungebrochenen Glasperlen kommt es zu einer Wechselwirkung zwischen der durch die gebrochenen Glasperlen eingekerbten Produktoberfläche und der durch die ungebrochenen Glasperlen eingeglätteten Produktoberfläche. Hierdurch können ebenfalls Materialdopplungen entstehen. Zusätzlich zur plastischen Verformung und der hiermit einhergehenden Erzeugung von Eigenspannungen kann ein Materialübertrag des Strahlmittels auf die Produktoberfläche stattfinden. Dieser Materialübertrag ist besonders stark im Bereich von Kerben, in welchen Materialanhäufungen der Glasperlen zurückbleiben können.

Alternativ zu der vorstehend am Beispiel von Glasperlen beschriebenen Strahlmittelbehandlung können die Oberflächen von medizintechnischen Produkten gebürstet werden. Hierzu können die Produktoberflächen mit Bürstscheiben, beispielsweise mit Hilfe eines scheibenförmig gestalteten Schleifvlieses oder von scheibenförmig angeordneten Nylonfasern mit Schleifpartikeln, bearbeitet werden. Auf den Bürstscheiben sind üblicherweise Aluminium- und/oder Siliziumoxidpartikel aufgebracht. Durch einen Bürstschritt erhöht sich zwar die Korrosionsbeständigkeit der Produktoberfläche im Vergleich zu einer mattierten Produktoberfläche, nachteilig ist jedoch, dass gebürstete Produktoberflächen ein stärkeres Reflexionsverhalten aufweisen als mattierte Produktoberflächen.

Es ist ferner bekannt, dass sich durch Materialdopplungen ausgebildete Mikrostrukturen oder Kerben auf einer Produktoberfläche sowie eine damit einhergehende Erzeugung oder Erhöhung von Eigenspannungen im Produkt nachteilig auf dessen Korrosionsbeständigkeit auswirken. Im Falle eines Materialübertrags, beispielsweise während eines Bandschleifens und/oder Mattierens, kommt hinzu, dass das übertragene Material zusätzliche Mikrostrukturen generiert sowie die Schwächung einer Passivierungsschicht bewirken kann.

Aus der US 2017/0114472 A1 ist ein Verfahren bekannt, bei welchem ein selektives elektrochemisches Ätzen der Oberfläche eines metallischen Objekts durchgeführt wird.

Ferner ist die Durchführung von elektrochemischem Ätzen zur Erzeugung mikrostrukturierter Implantatoberflächen für eine Wirkstoffbeschichtung bekannt (Stöver et al.: "Microstructuring of stainless steel implants by electrochemical etching", Journal of Material Science, Bd. 41, Nr. 17, 1. September 2006, Seiten 5569 - 5575).

Weiter ist ein Verfahren zur Verbesserung der Antioxidationseigenschaft von mit Chrom imprägniertem Stahl aus der FR 2 149 542 A1 bekannt.

Weiter ist die Verwendung von elektrochemischem Ätzen zur Kennzeichnung chirurgischer Instrumente bekannt (Anonymus: "Technologies for Marking Surgical Instruments Guidance Document", 1. Januar 2017, Seiten 1 - 8, XP055815653).

Die Dokumente SE 143 429 C1 und RU 2 046 158 C1 offenbaren jeweils ein Verfahren zur Herstellung von Bauteilen chirurgischer Instrumente, bei denen Edelstahl elektrochemisch geätzt wird.

Die DE 10 2004 044 738 A1 offenbart ein Verfahren zur Erzeugung einer Strukturierung von Metalloberflächen, wobei die Strukturierung der Metalloberfläche durch elektrochemisches Ätzen erzeugt wird.

Die US 2007/0193326 A1 offenbart ein Verfahren zum Herstellen einer chirurgischen Nadel, bei welchem die Nadel wenigstens abschnittsweise in ein Säurebad eingetaucht wird, um eine matte Oberfläche zu erzeugen.

### AUFGABE UND LÖSUNG

Der Erfindung liegt die Aufgabe zugrunde, ein Verfahren zum Herstellen eines chirurgischen Instruments oder eines Bauteils davon bereitzustellen, welches bei gattungsgemäßen Verfahren auftretende Nachteile wenigstens teilweise vermeidet und insbesondere zu einem chirurgischen Instrument oder Bauteil davon mit erhöhter Korrosionsbeständigkeit sowie verringertem Reflexionsverhalten führt.

Die Erfindung stellt sich des Weiteren die Aufgabe, ein entsprechendes chirurgisches Instrument oder Bauteil davon bereitzustellen.

Die oben genannten Aufgaben werden erfindungsgemäß gelöst durch ein Verfahren mit den Merkmalen des unabhängigen Anspruchs 1 sowie durch ein Produkt gemäß Anspruch 14. Bevorzugte Ausgestaltungen des Verfahrens und des Produkts sind Gegenstand der abhängigen Ansprüche sowie der Beschreibung. Der Wortlaut sämtlicher Ansprüche wird hiermit durch ausdrückliche Bezugnahme zum Inhalt der Beschreibung gemacht.

Der Ausdruck "medizintechnisches Produkt" bedeutet im Sinne der vorliegenden Erfindung ein chirurgisches Instrument oder ein Bauteil eines chirurgischen Instruments.

Unter dem Ausdruck "Legierung" soll im Sinne der vorliegenden Erfindung ein makroskopisch homogener metallischer Werkstoff aus wenigstens zwei Elementen (Komponenten) verstanden werden, von welchen wenigstens ein Element ein Metall ist. Demnach kann der Ausdruck "Legierung" im Sinne der vorliegenden Erfindung einen makroskopisch homogenen metallischen Werkstoff bedeuten, welcher aus wenigstens zwei verschiedenen Metallen besteht. Alternativ kann der Ausdruck "Legierung" im Sinne der vorliegenden Erfindung einen makroskopisch homogenen metallischen Werkstoff bedeuten, welcher aus wenigstens einem Metall sowie wenigstens einem Nichtmetall, wie beispielsweise Kohlenstoff, besteht.

Überraschenderweise stellte sich heraus, dass sich die eingangs im Zusammenhang von konventionellen Oberflächenbehandlungen von medizintechnischen Produkten auftretenden Nachteile teilweise oder sogar vollständig durch ein elektrochemisches Ätzen von medizintechnischen Produkten vermeiden lassen. So konnte am Beispiel von chirurgischen Instrumenten gezeigt werden, dass ein elektrochemisches Ätzen eine Verringerung der Lichtreflexion an der Produktoberfläche und außerdem eine Erhöhung der Korrosionsbeständigkeit bewirkt. Beim verwendeten chromhaltigen oder chromlegierten Edelstahl beruht die erhöhte Korrosionsbeständigkeit insbesondere darauf, dass infolge des elektrochemischen Ätzvorgangs sechswertige Chromionen in Lösung gehen. Dies hat zur Folge, dass chromreiche Oxidschichten an der Oberfläche des medizintechnischen Produkts entfernt werden, wodurch ein direkter Angriff einer für den elektrochemischen Ätzvorgang verwendeten Säure auf chemische und physikalische Inhomogenitäten, wie chromcarbidhaltige Bereiche um Chromcarbide, der Oberfläche des medizintechnischen Produkts ermöglicht wird. Dadurch entsteht, insbesondere an Stellen ehemaliger Chromcarbid-Bereiche, eine Mikrostruktur, insbesondere mit oder in Form von vorzugsweise offenen Ätzgrübchen, auf der Oberfläche des medizintechnischen Produkts. Zusätzlich kann vorteilhafterweise eine Auflösung von Grenzbereichen, insbesondere von Latten- und Subblockgrenzen, erfolgen, die insbesondere ein Herausstechen von einzelnen Martensitlatten zur Folge haben kann. Das Resultat ist eine aufgeraute Produktoberfläche, an welcher einfallendes Licht gestreut werden kann. Dadurch wirkt die Oberfläche des medizintechnischen Produkts matt, wodurch sich mit besonderem Vorteil die Handhabung des medizintechnischen Produkts für einen Anwender vereinfacht. Beispielsweise kann hierdurch ein Blenden eines Chirurgen im Operationssaal vermieden werden. Durch den Abbau chromverarmter Bereiche auf der Oberfläche des medizintechnischen Produkts wird ferner vorteilhafterweise das Risiko der Entstehung von Keimstellen einer Lochkorrosion gesenkt.

Ein weiterer Vorteil besteht darin, dass durch den elektrochemischen Ätzvorgang die Ausbildung einer Passivschicht, insbesondere einer im Vergleich zum Stand der Technik dickeren Passivschicht, begünstigt werden kann. Dadurch kann die Korrosionsbeständigkeit des medizintechnischen Produkts zusätzlich erhöht werden.

Ein weiterer Vorteil des elektrochemischen Ätzens besteht insbesondere darin, dass Druck- und Zugspannungen und/oder Materialdopplungen und/oder Materialüberlappungen auf der Oberfläche des medizintechnischen Produkts weitgehend oder vollständig vermieden werden können. Dadurch kann das Korrosionsrisiko zusätzlich reduziert werden.

Ferner können durch das elektrochemische Ätzen vorteilhafterweise etwaige korrosionsauslösende Materialfehler auf der Oberfläche des medizintechnischen Produkts abgebaut werden.

Ferner führt das erfindungsgemäße Verfahren gegenüber gattungsgemäßen Verfahren vorteilhafterweise zu vergleichbar oder besser zu reinigenden Oberflächen des medizintechnischen Produkts und/oder zu einer vergleichbaren oder besseren Kratzbeständigkeit des medizintechnischen Produkts und/oder zu einer vergleichbaren oder besseren mechanischen Beständigkeit des medizintechnischen Produkts und/oder zu einer vergleichbaren oder besseren Haptik, insbesondere Glattheit, des medizintechnischen Produkts.

In Ausgestaltung der Erfindung wird vor dem Durchführen von Schritt a) ein Schleifen, vorzugsweise ein Gleit- und/oder Bandschleifen, der Oberfläche des medizintechnischen Produkts durchgeführt.

Zum Gleitschleifen wird das medizintechnische Produkt bevorzugt zusammen mit Gleitschleifkörpern, welche vorzugsweise als Schüttgut gestaltet sind, oder zusammen mit einer Gleitschleifkörper und optional Zusatzstoffe enthaltenden wässrigen Lösung in einen Behälter gegeben. Die optional vorgesehenen Zusatzstoffe können ausgewählt sein aus der Gruppe bestehend aus Korrosionsschutzmittel, Entfettungsmittel, Beizmittel, Trennmittel (beispielsweise Kunststoffkügelchen mit einem Durchmesser < 1 mm) und Mischungen davon. Durch eine solche Lösung lassen sich in vorteilhafter Weise ein durch die Gleitschleifkörper entstehender Abrieb sowie ein Produktabtrag aufnehmen und abtransportieren. Abhängig vom jeweils verwendeten Zusatzstoff lassen sich darüber hinaus weitere Effekte realisieren, wie beispielsweise Korrosionsschutz, Entfettung sowie Adhäsionsprophylaxe.

Durch eine oszillierende oder rotierende Bewegung des Behälters entsteht eine Relativbewegung zwischen dem medizintechnischen Produkt und den Gleitschleifkörpern. Dadurch wird ein Materialabtrag am medizintechnischen Produkt, insbesondere an dessen Kanten, hervorgerufen. Das Oberflächenbild des medizintechnischen Produkts, die Rauheit, der Materialabtrag sowie die Entgratleistung lassen sich in vorteilhafter Weise gezielt durch zum Gleitschleifen verwendete Maschinen, Schleifkörper sowie durch optionale Zusatzmittel beeinflussen.

Die Gleitschleifkörper können ein Material aufweisen oder aus einem Material bestehen, welches ausgewählt ist aus der Gruppe bestehend aus Keramik, Kunststoff, Naturprodukt wie Walnussschalen, Stahl und Kombinationen davon.

Grundsätzlich können die Gleitschleifkörper regelmäßig und/oder unregelmäßig geformt vorliegen.

Die Gleitschleifkörper können insbesondere ecken- und/oder kantenfrei, wie beispielsweise ellipsoid-, toroid- oder kugelförmig, gestaltet sein.

Alternativ oder in Kombination können die Gleitschleifkörper Ecken und/oder Kanten aufweisen. Insbesondere können die Gleitschleifkörper polyederförmig, beispielsweise würfelförmig, quaderförmig, prismenförmig, pyramidenförmig oder spatförmig, gestaltet sein. Weiterhin können die Gleitschleifkörper insbesondere als gerade Prismen und/oder schiefe Prismen gestaltet sein.

Alternativ oder in Kombination können die Gleitschleifkörper kegel- und/oder kegelstumpfförmig gestaltet sein.

Ferner kann eine Mischung unterschiedlich gestalteter Gleitschleifkörper zum Gleitschleifen des medizintechnischen Produkts verwendet werden. Beispielsweise können ecken- und/oder kantenfreie Gleitschleifkörper und polyederförmige Gleitschleifkörper verwendet werden. Alternativ oder in Kombination können unterschiedlich gestaltete ecken- und/oder kantenfreie Gleitschleifkörper und/oder unterschiedliche polyederförmige Gleitschleifkörper verwendet werden. Bezüglich in Frage kommender Gestaltungen und Formen wird vollständig auf die in den vorherigen Absätzen beschriebenen Gestaltungen und Formen für die Gleitschleifkörper Bezug genommen.

Die Gleitschleifkörper können ferner wenigstens eine Abmessung, insbesondere wenigstens eine mittlere Abmessung, wie beispielsweise einen Durchmesser, insbesondere mittleren Durchmesser, und/oder eine Höhe, insbesondere mittlere Höhe, und/oder eine Länge, insbesondere mittlere Länge, im Bereich von 1 mm bis 80 mm aufweisen. Dabei soll unter dem Durchmesser von kugelförmig gestalteten Gleitschleifkörpern im Sinne der vorliegenden Erfindung der doppelte Radius eines einzelnen kugelförmig gestalteten Gleitschleifkörpers verstanden werden. Dagegen soll unter dem Durchmesser eines nicht kugelförmig gestalteten Gleitschleifkörpers im Sinne der vorliegenden Erfindung der größtmögliche Abstand zweier Punkte verstanden werden, welchen diese entlang einer Umfangslinie eines einzelnen, nicht kugelförmig gestalteten Gleitschleifkörpers zueinander einnehmen können. Die in diesem Absatz genannten mittleren Abmessungen können beispielsweise mittels Schüttdichte und/oder optischer Vermessung bestimmt werden. Das Gleitschleifen kann weiterhin als Trommelgleitschleifen, Vibrationsgleitschleifen, Tauchgleitspanen, Schleppschleifen, Fliehkraftgleitspanen oder Druckfließläppen durchgeführt werden.

Zum Bandschleifen des medizintechnischen Produkts werden vorzugsweise Schleifbänder verwendet. Hierfür können insbesondere Schleifbänder verwendet werden, welche über wenigstens zwei Rollen umlaufen. Die Schleifbänder weisen bevorzugt eine Körnung von 150 bis 1.200 auf. Die Zahl der Körnung orientiert sich dabei an der Maßeinheit Mesh, d.h. der Anzahl der Maschen eines Netzes pro Zoll (25,4 mm). Demzufolge passt beispielsweise ein Schleifmittel mit der Körnung 150 gerade noch durch ein Sieb mit 150 Maschen pro Zoll.

Erfindungsgemäß kann vor dem Durchführen von Schritt a) beispielsweise zunächst ein Gleitschleifen und anschließend ein Bandschleifen durchgeführt werden. Ein Bandschleifen kann vor allem im Hinblick auf das Behandeln eines sogenannten Abschattungsbereichs des medizintechnischen Produkts, aber auch außerhalb eines solchen Bereichs von Vorteil sein. Der Abschattungsbereich definiert den Bereich eines medizintechnischen Produkts, in welchem Gleitschleifkörper, insbesondere aufgrund der geometrischen Form und/oder Gestalt des medizintechnischen Produkts, nicht oder nur begrenzt auf der Oberfläche wirksam sind.

Alternativ kann die Oberfläche des medizintechnischen Produkts vor dem Durchführen von Schritt a) lediglich mittels Gleitschleifen geschliffen werden. Dadurch kann die Entstehung von auf Bandschleifen zurückgehende Kerben und/oder Anhebungen auf der Produktoberfläche vermieden und mithin die Korrosionsbeständigkeit des medizintechnischen Produkts zusätzlich verbessert werden.

Alternativ kann die Oberfläche des medizintechnischen Produkts vor dem Durchführen von Schritt a) lediglich mittels Bandschleifen geschliffen werden.

In weiterer Ausgestaltung der Erfindung wird die Oberfläche des medizintechnischen Produkts nicht mit einem Strahlmittel behandelt. Wie bereits erwähnt, bewirkt der erfindungsgemäß vorgesehene Ätzschritt bereits vorteilhafterweise eine Mattierung der Oberfläche des medizintechnischen Produkts, weswegen eine Mattierung durch Behandeln mit einem Strahlmittel entbehrlich ist. Dadurch lassen sich mit besonderem Vorteil Bearbeitungs-/Herstellungszeiten und/oder -kosten für das medizintechnische Produkt signifikant reduzieren. Ferner lässt sich hierdurch das Risiko eines Materialübertrags von einem Strahlmittel auf das medizintechnische Produkt vermeiden, wodurch dessen Korrosionsbeständigkeit zusätzlich verbessert werden kann.

Alternativ kann die Oberfläche des medizintechnischen Produkts, vorzugsweise vor dem Durchführen von Schritt a), insbesondere zwischen einem Schleifen, insbesondere Gleit- und/oder Bandschleifen, der Oberfläche des medizintechnischen Produkts und dem Durchführen von Schritt a), mit einem Strahlmittel behandelt werden. Als Strahlmittel kann insbesondere ein duktiles, d.h. nicht sprödes, Strahlmittel verwendet werden. Durch die Verwendung eines derartigen Strahlmittels kann mit besonderem Vorteil die Erzeugung von Kerben und/oder von Mikrostrukturen, insbesondere in Form von Mikrospalten, auf der Oberfläche des medizintechnischen Produkts verhindert oder wenigstens reduziert werden. Dadurch kann das Auftreten lokaler Spannungsspitzen im medizintechnischen Produkt vermieden oder wenigstens reduziert und insbesondere die Korrosionsbeständigkeit des medizintechnischen Produkts zusätzlich verbessert werden. Obendrein lässt sich durch die Verwendung eines derartigen Strahlmittels in vorteilhafter Weise die Kratzbeständigkeit des medizintechnischen Produkts verbessern. Bezüglich des in diesem Absatz erwähnten Schleifens, insbesondere Gleit- und/oder Bandschleifens, der Oberfläche des medizintechnischen Produkts wird vollständig auf die entsprechenden in der bisherigen Beschreibung gemachten Ausführungen Bezug genommen.

Grundsätzlich kann das Strahlmittel ein Material aufweisen oder aus einem Material bestehen, welches ausgewählt ist aus der Gruppe bestehend aus Metall, Metalloxid, Legierung, Keramik, Kunststoff, pflanzlicher Stoff, Sand und Kombinationen davon.

Bei dem Metall kann es sich insbesondere um Aluminium handeln.

Bei dem Metalloxid kann es sich insbesondere um Aluminiumoxid (Al₂O₃), vorzugsweise des Typs Korund, handeln.

Bei dem Kunststoff kann es sich insbesondere um ein Harnstoff-, Phenol-, Polyester- oder Melamin-Harz handeln.

Bei der Keramik kann es sich insbesondere um Glas oder eine Mischkeramik handeln.

Bei der Legierung kann es sich zum Beispiel um Stahl, insbesondere Edelstahl, handeln. Bevorzugt handelt es sich bei der Legierung um einen nichtrostenden Stahl, insbesondere nichtrostenden Edelstahl. Bezüglich geeigneter Edelstähle sei auf die noch folgende Beschreibung Bezug genommen.

Bei dem Sand kann es sich insbesondere um Granatsand handeln.

Bevorzugt weist das Strahlmittel ein Metall oder eine Legierung auf oder bevorzugt besteht das Strahlmittel aus einem Metall oder einer Legierung. Ein solches Strahlmittel hat insbesondere den Vorteil, dass es nicht bricht und mithin keine Einkerbung der Oberfläche des medizintechnischen Produkts verursacht. Obendrein kann ein Materialübertrag auf die Produktoberfläche reduziert oder sogar vollständig vermieden werden. Insgesamt kann dadurch die Korrosionsbeständigkeit des medizintechnischen Produkts zusätzlich verbessert sowie das Auftreten unerwünschter Eigenspannungen im Produkt vermieden werden. Außerdem ist ein solches Strahlmittel besonders geeignet, die Kratzbeständigkeit des medizintechnischen Produkts zu erhöhen.

Bevorzugt weist das Strahlmittel Stahl, insbesondere Edelstahl, auf oder bevorzugt besteht das Strahlmittel aus Stahl, insbesondere Edelstahl. Durch ein solches Strahlmittel können die im letzten Absatz erwähnten Vorteile besonders stark zur Geltung gebracht werden.

Grundsätzlich kann das Strahlmittel regelmäßig und/oder unregelmäßig geformt, insbesondere als regelmäßig und/oder unregelmäßig geformte Strahlmittelkörper, vorliegen.

Ferner ist es bevorzugt, dass das Strahlmittel ecken- und/oder kantenfrei, insbesondere als ecken- und/oder kantenfreie Strahlmittelkörper, gestaltet ist. Dadurch kann die Erzeugung von Einkerbungen auf der Oberfläche des medizintechnischen Produkts vermieden und mithin dessen Korrosionsbeständigkeit zusätzlich verbessert werden.

Grundsätzlich kann das Strahlmittel ellipsoid-, toroid-, kugel- oder perlenförmig gestaltet sein oder in Form entsprechend gestalteter Strahlmittelkörper vorliegen.

Bevorzugt ist das Strahlmittel kugel- und/oder perlenförmig oder als kugel- und/oder perlenförmige Strahlmittelkörper gestaltet.

Alternativ oder in Kombination kann das Strahlmittel Ecken und/oder Kanten aufweisen. Insbesondere kann das Strahlmittel polyederförmig, beispielsweise würfelförmig, quaderförmig, prismenförmig, pyramidenförmig oder spatförmig, gestaltet sein oder als entsprechend gestaltete Strahlmittelkörper vorliegen. Das Strahlmittel kann ferner die Form eines geraden Prismas oder schiefen Prismas aufweisen oder in Form entsprechend gestalteter Strahlmittelkörper vorliegen.

Alternativ oder in Kombination kann das Strahlmittel kegel- und/oder kegelstumpfförmig gestaltet sein oder in Form von kegel- und/oder kegelstumpfförmigen Strahlmittelkörpern vorliegen.

Alternativ oder in Kombination kann das Strahlmittel in globularer Form, beispielsweise in Form eines arrondierten Drahts, oder in Form entsprechend gestalteter Strahlmittelkörper vorliegen.

Alternativ oder in Kombination kann das Strahlmittel in gebrochener Form, insbesondere in Form von gebrochenen Schleifmittelkörpern, vorliegen.

Ferner kann/können das Strahlmittel oder die Strahlmittelkörper wenigstens eine Abmessung, insbesondere wenigstens eine mittlere Abmessung, wie beispielsweise einen Durchmesser, insbesondere mittleren Durchmesser, und/oder eine Höhe, insbesondere mittlere Höhe, und/oder eine Länge, insbesondere mittlere Länge, im Bereich von 40 µm bis 2000 µm aufweisen. Dabei soll unter dem Durchmesser eines kugelförmig gestalteten Strahlmittels oder von kugelförmig gestalteten Strahlmittelkörpern im Sinne der vorliegenden Erfindung der doppelte Radius eines kugelförmig gestalteten Strahlmittels bzw. eines einzelnen kugelförmig gestalteten Strahlmittelkörpers verstanden werden. Dagegen soll unter dem Durchmesser eines nicht kugelförmig gestalteten Strahlmittels oder von nicht kugelförmig gestalteten Strahlmittelkörpern im Sinne der vorliegenden Erfindung der größtmögliche Abstand zweier Punkte verstanden werden, welchen diese entlang einer Umfangslinie eines nicht kugelförmig gestalteten Strahlmittels bzw. eines einzelnen nicht kugelförmig gestalteten Strahlmittelkörpers zueinander einnehmen können. Die in diesem Absatz genannten mittleren Abmessungen können beispielsweise mittels Laserbeugung oder Siebanalyse bestimmt werden.

Zur Beschleunigung des Strahlmittels oder der Strahlmittelkörper auf die Oberfläche des medizintechnischen Produkts können beispielsweise Druckstrahlanlagen, Injektorstrahlanlagen oder Schleuderradanlagen eingesetzt werden. Kommt eine Druckstrahl- oder Injektorstrahlanlage zum Einsatz, können Drücke von 1 bar bis 6 bar verwendet werden.

In weiterer Ausgestaltung der Erfindung wird die Oberfläche des medizintechnischen Produkts nicht elektropoliert.

Alternativ kann die Oberfläche des medizintechnischen Produkts, insbesondere vor dem Durchführen von Schritt a), insbesondere zwischen einem Schleifen, insbesondere Gleit- und/oder Bandschleifen der Oberfläche des medizintechnischen Produkts und dem Durchführen von Schritt a), insbesondere zwischen einem Behandeln der Oberfläche des medizintechnischen Produkts mit einem Strahlmittel und dem Durchführen von Schritt a), und/oder nach dem Durchführen von Schritt a), insbesondere zwischen dem Durchführen von Schritt a) und einem Behandeln der Oberfläche des medizintechnischen Produkts mit einer Passiviersäure oder einer passiviersäurehaltigen Lösung, elektropoliert werden. Zum Durchführen des Elektropolierens wird in der Regel eine wässrige Elektrolytlösung verwendet. Vorzugsweise weist die wässrige Elektrolytlösung eine Mineralsäure oder ein Mineralsäuregemisch, insbesondere ausgewählt aus der Gruppe bestehend aus Phosphorsäure, Schwefelsäure und ein Gemisch davon, auf. Die wässrige Elektrolytlösung kann ferner einen Phosphorsäureanteil von 20 Gew.-% bis 70 Gew.-%, insbesondere 30 Gew.-% bis 60 Gew.-%, bevorzugt 40 Gew.-% bis 50 Gew.-%, bezogen auf das Gesamtgewicht der wässrigen Elektrolytlösung, und/oder oder einen Schwefelsäureanteil von 10 Gew.-% bis 70 Gew.-%, insbesondere 20 Gew.-% bis 60 Gew.-%, bevorzugt 30 Gew.-% bis 50 Gew.-%, bezogen auf das Gesamtgewicht der wässrigen Elektrolytlösung, aufweisen. Ferner ist es bevorzugt, wenn die Oberfläche des medizintechnischen Produkts bei einer Spannung, insbesondere Gleichspannung, von 2 V bis 10 V elektropoliert wird. Dabei kann die Spannung während des Elektropolierens konstant gehalten oder variiert werden. Ferner ist es bevorzugt, wenn die Oberfläche des medizintechnischen Produkts bei einer Stromdichte von 5 A/dm² bis 50 A/dm² elektropoliert wird. Ferner kann es bevorzugt sein, wenn die Oberfläche des medizintechnischen Produkts bei einer Temperatur von 50 °C bis 65 °C elektropoliert wird. Bezüglich des in diesem Absatz erwähnten Schleifens, insbesondere Gleit- und/oder Bandschleifens, der Oberfläche des medizintechnischen Produkts sowie des Behandelns der Oberfläche des medizintechnischen Produkts mit einem Strahlmittel wird vollständig auf die entsprechenden in der bisherigen Beschreibung gemachten Ausführungen Bezug genommen. Bezüglich des in diesem Absatz erwähnten Behandelns der Oberfläche des medizintechnischen Produkts mit einer Passiviersäure oder einer passiviersäurehaltigen Lösung wird vollständig auf die entsprechenden in der noch folgenden Beschreibung gemachten Ausführungen Bezug genommen.

Üblicherweise wird zum Durchführen von Schritt a) die Oberfläche des medizintechnischen Produkts in einer Elektrolytlösung anodisch abgetragen, d.h. das medizintechnische Produkt bildet die Anode in einer elektrochemischen Zelle.

In weiterer Ausgestaltung der Erfindung wird der Schritt a) mehrmals, insbesondere zwei-, drei- oder viermal, durchgeführt.

Hierdurch lassen sich mit besonderem Vorteil geometrische Besonderheiten des medizintechnischen Produkts, wie zum Beispiel der Schluss des medizintechnischen Produkts, gleichmäßig bearbeiten, ohne dass eine relevante Abschattung entsteht. Der Schluss des medizintechnischen Produkts kann in zwei Positionen bearbeitet werden, damit nur geringe Abschattungen entstehen. Alternativ kann es bevorzugt sein, dass das medizintechnische Produkt während des Durchführens von Schritt a) langsam artikuliert wird.

Alternativ kann der Schritt a) nur einmal durchgeführt werden.

In weiterer Ausgestaltung der Erfindung wird zum Durchführen von Schritt a) eine saure, wässrige Elektrolytlösung, insbesondere aufweisend eine Mineralsäure oder ein Mineralsäuregemisch, verwendet.

In weiterer Ausgestaltung der Erfindung wird die Mineralsäure ausgewählt aus der Gruppe bestehend aus Phosphorsäure, Schwefelsäure und ein Gemisch davon. Eine phosphorsäure- und/oder schwefelsäurehaltige, wässrige Elektrolytlösung hat sich dabei als besonders vorteilhaft für ein elektrochemisches Ätzen der Oberfläche eines medizintechnischen Produkts aus Edelstahl, insbesondere korrosionsbeständigem Edelstahl, herausgestellt.

Bei der sauren, wässrigen Elektrolytlösung kann es sich ferner um eine gealterte, saure, wässrige Elektrolytlösung handeln.

Ferner kann die saure, wässrige Elektrolytlösung einen Mineralsäureanteil von 50 Gew.-% bis 95 Gew.-%, insbesondere 60 Gew.-% bis 95 Gew.-%, vorzugsweise 75 Gew.-% bis 95 Gew.-%, aufweisen, bezogen auf das Gesamtgewicht der sauren, wässrigen Elektrolytlösung. Insbesondere kann die saure, wässrige Elektrolytlösung einen Phosphorsäureanteil von 10 Gew.-% bis 70 Gew.-%, insbesondere 20 Gew.-% bis 70 Gew.-%, insbesondere 30 Gew.-% bis 60 Gew.-%, vorzugsweise 40 Gew.-% bis 50 Gew.-%, und/oder einen Schwefelsäureanteil von 10 Gew.-% bis 70 Gew.-%, insbesondere 20 Gew.-% bis 60 Gew.-%, vorzugsweise 30 Gew.-% bis 50 Gew.-%, aufweisen, jeweils bezogen auf das Gesamtgewicht der sauren, wässrigen Elektrolytlösung.

Die saure, wässrige Elektrolytlösung kann ferner Zusatzstoffe, wie beispielsweise oberflächenaktive Substanzen, aufweisen.

Von Vorteil ist, dass sich die Aggressivität der sauren, wässrigen Elektrolytlösung gezielt über deren Wasseranteil steuern lässt. Beispielsweise kann die saure, wässrige Elektrolytlösung einen Wasseranteil von 5 Gew.-% bis 25 Gew.-%, insbesondere 5 Gew.-% bis 15 Gew.-%, vorzugsweise 5 Gew.-% bis 10 Gew.-%, aufweisen, bezogen auf das Gesamtgewicht der sauren, wässrigen Elektrolytlösung.

In weiterer Ausgestaltung der Erfindung wird der Schritt a) während eines Zeitraumes von 6 min bis 14 min, insbesondere von 8 min bis 12 min, vorzugsweise von 10 min, durchgeführt.

Der Schritt a) wird bei einer an der Anode (an dem zu oberflächenbehandelnden oder - bearbeitenden und/oder herzustellenden medizintechnischen Produkt) gemessenen Spannung, insbesondere Gleichspannung, von 1,4 V bis 1,7 V, besonders bevorzugt 1,4 V bis 1,5 V oder 1,45 V bis 1,65 V, durchgeführt. Bei dieser Ausgestaltung der Erfindung kommen die erfindungsgemäßen Vorteile besonders stark zur Geltung. Die Messung der Spannung an der Anode (an dem zu oberflächenbehandelnden oder -bearbeitenden und/oder herzustellenden medizintechnischen Produkt) erfolgt vorzugsweise über eine Silber-Silberchlorid-Elektrode. Die ermittelten Spannungen werden anschließend auf eine Standardwasserstoffelektrode umgerechnet. Üblicherweise wird die Spannung an der Stromquelle eingestellt, ohne zu wissen, welcher Teil der Spannung an der Anode anliegt und wie viel an restlichen Widerständen (zum Beispiel Leitungen, Elektrolyt, usw.) anliegt. Bei der vorliegenden Erfindung ist vorzugsweise die genaue Spannung an der Anode entscheidend.

Ferner kann der Schritt a) bei/mit einer konstanten oder variierenden Spannung, insbesondere Gleichspannung, durchgeführt werden. Bezüglich geeigneter Spannungsbereiche/-werte wird auf die im vorangegangenen Absatz offenbarten Spannungen Bezug genommen.

Alternativ oder in Kombination wird der Schritt a) bei einer Stromdichte von 1,6 A/dm² bis 2,2 A/dm², vorzugweise 1,8 A/dm² bis 2,0 A/dm², durchgeführt. Durch die in diesem Absatz offenbarten (niedrigen) Stromdichten lässt sich ein Ätzen der Oberfläche des medizintechnischen Produkts in zeitlicher Hinsicht besonders gut steuern.

In weiterer Ausgestaltung der Erfindung wird der Schritt a) bei einer Temperatur von 20 °C bis 90 °C, insbesondere 50 °C bis 80 °C, vorzugweise 70 °C bis 80 °C, durchgeführt.

In weiterer Ausgestaltung der Erfindung wird die Oberfläche des medizintechnischen Produkts, insbesondere nach Durchführen von Schritt a), nicht mit einer Passiviersäure oder einer passiviersäurehaltigen Lösung behandelt. Wie bereits erwähnt, kann nämlich bereits der erfindungsgemäß vorgesehene Ätzschritt (Schritt a)) mit besonderem Vorteil die Ausbildung einer Passivschicht auf der Oberfläche des medizintechnischen Produkts begünstigen und mithin eine Verbesserung der Korrosionsbeständigkeit des medizintechnischen Produkts bewirken. Diese Ausgestaltung der Erfindung hat (ebenfalls) den Vorteil einer deutlichen Verringerung der Bearbeitungs-/Herstellungszeiten und/oder -kosten für das medizintechnische Produkt.

Alternativ kann die Oberfläche des medizintechnischen Produkts, insbesondere nach Durchführen von Schritt a), insbesondere nach einem Elektropolieren der Oberfläche des medizintechnischen Produkts, mit einer Passiviersäure oder einer passiviersäurehaltigen Lösung, insbesondere passiviersäurehaltigen, wässrigen Lösung, behandelt werden. Bezüglich des in diesem Absatz erwähnten Elektropolierens der Oberfläche des medizintechnischen Produkts wird vollständig auf die entsprechenden in der bisherigen Beschreibung gemachten Ausführungen Bezug genommen.

Hierdurch kann die Ausbildung einer Passivschicht auf der Oberfläche des medizintechnischen Produkts zusätzlich verstärkt oder begünstigt und mithin die Korrosionsbeständigkeit des medizintechnischen Produkts zusätzlich verbessert werden. Im Fall eines medizintechnischen Produkts aus einem chromhaltigen oder chromlegierten Edelstahl lassen sich durch einen Passivierungsschritt beispielsweise verstärkte Chromoxidschichten auf der Oberfläche des medizintechnischen Produkts ausbilden.

Als Passiviersäuren können beispielsweise Zitronensäure und/oder Salpetersäure verwendet werden. Als passiviersäurehaltige Lösung kann beispielsweise eine wässrige, zitronensäurehaltige Lösung, insbesondere mit einem Zitronensäureanteil von 5 Gew.-% bis 60 Gew.-%, bezogen auf das Gesamtgewicht der wässrigen, zitronensäurehaltigen Lösung, verwendet werden. Alternativ kann als passiviersäurehaltige Lösung eine wässrige, salpetersäurehaltige Lösung, insbesondere mit einem Salpetersäureanteil von 5 Gew.-% bis 60 Gew.-%, bezogen auf das Gesamtgewicht der wässrigen, salpetersäurehaltigen Lösung, verwendet werden.

Die Verwendung von Zitronensäure hat sowohl unter Gesundheits- als auch Arbeitssicherheitsgesichtspunkten Vorteile gegenüber einer Verwendung von Salpetersäure. Obendrein lassen sich mittels Zitronensäure im Fall von medizintechnischen Produkten aus chromhaltigem oder chromlegiertem Edelstahl dickere Chromoxidschichten realisieren, als dies bei Verwendung von Salpetersäure der Fall ist, da Letztere im Fall eines solchen Edelstahls auch den Anteil anderer Legierungsbestandteile reduziert.

Zum Durchführen der Passivierung kann das medizintechnische Produkt beispielsweise in die Passiviersäure oder die passiviersäurehaltige Lösung eingetaucht werden. Alternativ kann die Passiviersäure oder die passiviersäurehaltige Lösung auf die Oberfläche des medizintechnischen Produkts gesprüht oder gegossen werden.

Ferner kann die Oberfläche des medizintechnischen Produkts während eines Zeitraumes von 2 min bis 120 min, insbesondere 5 min bis 60 min, bevorzugt 10 min bis 30 min, mit der Passiviersäure oder passiviersäurehaltigen Lösung behandelt werden.

Ferner kann Oberfläche des medizintechnischen Produkts in einem Temperaturbereich von 20 °C bis 80 °C, insbesondere 30 °C bis 65 °C, bevorzugt 50 °C bis 60 °C, mit der Passiviersäure oder passiviersäurehaltigen Lösung behandelt werden.

Ferner kann zwischen dem Schritt a) und dem Behandeln der Oberfläche des medizintechnischen Produkts mit der Passiviersäure oder passiviersäurehaltigen Lösung, insbesondere zwischen einem Elektropolieren der Oberfläche des medizintechnischen Produkts und dem Behandeln der Oberfläche des medizintechnischen Produkts mit der Passiviersäure oder passiviersäurehaltigen Lösung, ein Reinigen und/oder Entfetten der Oberfläche des medizintechnischen Produkts durchgeführt werden. Bezüglich des in diesem Absatz erwähnten Elektropolierens der Oberfläche des medizintechnischen Produkts wird vollständig auf die entsprechenden in der bisherigen Beschreibung gemachten Ausführungen Bezug genommen.

In weiterer Ausgestaltung der Erfindung wird nach dem Durchführen von Schritt a), insbesondere nach einem Elektropolieren der Oberfläche des medizintechnischen Produkts, insbesondere nach einem Behandeln der Oberfläche des medizintechnischen Produkts mit einer Passiviersäure oder einer passiviersäurehaltigen Lösung, ein Schritt b) Verpacken und/oder Kennzeichnen, insbesondere Etikettieren, des medizintechnischen Produkts durchgeführt. Bevorzugt wird zwischen dem Schritt a) und dem Schritt b), insbesondere zwischen einem Elektropolieren der Oberfläche des medizintechnischen Produkts und dem Schritt b), insbesondere zwischen einem Behandeln der Oberfläche des medizintechnischen Produkts mit einer Passiviersäure oder einer passiviersäurehaltigen Lösung, ein Schritt ab) Sterilisieren, insbesondere Dampfsterilisieren, des medizintechnischen Produkts durchgeführt. Alternativ kann es bevorzugt sein, dass nach dem Durchführen von Schritt b) ein Schritt c) Sterilisieren, insbesondere Dampfsterilisieren, des medizintechnischen Produkts durchgeführt wird. Bezüglich des in diesem Absatz erwähnten Elektropolierens der Oberfläche des medizintechnischen Produkts sowie des in diesem Absatz erwähnten Behandelns der Oberfläche des medizintechnischen Produkts mit einer Passiviersäure oder einer passiviersäurehaltigen Lösung wird vollständig auf die entsprechenden in der bisherigen Beschreibung gemachten Ausführungen Bezug genommen.

Das medizintechnische Produkt weist chromhaltigen Edelstahl auf oder besteht daraus.

Unter dem Ausdruck "Edelstahl" soll im Sinne der vorliegenden Erfindung (in Übereinstimmung nach EN 10020) ein legierter Stahl mit besonderem Reinheitsgrad, beispielsweise mit einem Schwefel- und/oder Phosphormassenanteil ≤ 0,025 %, insbesondere < 0,025 %, verstanden werden.

Neben Chrom kann der Edelstahl wenigstens ein Legierungselement, ausgewählt aus der Gruppe bestehend aus Nickel, Molybdän, Titan, Niob, Wolfram, Vanadium, Kobalt und Kombinationen davon, aufweisen.

Insbesondere kann der Edelstahl einen Chrommassenanteil von 10 % bis 25 % aufweisen. Weiter bevorzugt handelt es sich bei dem Edelstahl um einen nichtrostenden oder korrosionsbeständigen Edelstahl.

Bei dem Edelstahl handelt es sich um einen chromhaltigen oder chromlegierten Edelstahl. Vorzugsweise handelt es sich bei dem Edelstahl um einen chromhaltigen, korrosionsbeständigen Edelstahl oder um einen chromlegierten, korrosionsbeständigen Edelstahl.

Ferner kann es sich bei dem Edelstahl insbesondere um einen martensitischen, ferritischen oder austenitischen Edelstahl handeln.

Vorzugsweise handelt es sich bei dem Edelstahl um einen martensitischen, korrosionsbeständigen Edelstahl, insbesondere um einen sogenannten Kohlenstoffmartensit, d.h. einen korrosionsbeständigen Edelstahl mit Chrom und Kohlenstoff als Hauptlegierungsbestandteile, oder um einen sogenannten Nickelmartensit, d.h. um einen korrosionsbeständigen Edelstahl mit Nickel als Hauptlegierungsbestandteil, gemäß ISO 7153-1. Insbesondere kann es sich bei dem Edelstahl um einen martensitischen Edelstahl mit einem Chrommassenanteil von 10,5 % bis 13 % und/oder einem Kohlenstoffmassenanteil von 0,2 % bis 1 % handeln.

Alternativ kann es sich bei dem Edelstahl insbesondere um einen austenitischen Edelstahl mit einem Chrommassenanteil von 16 % bis 21 % und/oder einem Kohlenstoffmassenanteil von 0,02 % bis 0,12 % handeln.

Alternativ kann es sich bei dem Edelstahl insbesondere um einen ferritischen Edelstahl mit einem Chrommassenanteil von 12 % bis 18 % und/oder einem Kohlenstoffmassenanteil von < 0,2 % handeln.

Beispielsweise kann es sich bei dem Edelstahl um einen Edelstahl mit der Werkstoffkurzbezeichnung X12Cr13 (Werkstoffnummer 1.4006) handeln. Hierbei handelt es sich um einen martensitischen Edelstahl mit einem Kohlenstoffmassenanteil von 0,08 % bis 0,15 %, einem Chrommassenanteil von 11,5 % bis 13,5 % sowie einem Nickelmasseanteil von ≤ 0,75 %.

Alternativ kann es sich bei dem Edelstahl um einen martensitischen, korrosionsbeständigen Edelstahl mit der Werkstoffkurzbezeichnung X12CrS13 (Werkstoffnummer 1.4005) handeln. Dieser Edelstahl weist einen Kohlenstoffmassenanteil von 0,08 % bis 0,15 %, einen Chrommassenanteil von 12,0 % bis 14,0 % sowie eine Molybdänmassenanteil ≤ 0,60 % sowie optional einen Schwefelmassenanteil von 0,15 % bis 0,35 % auf.

Alternativ kann es sich bei dem Edelstahl um einen martensitischen, korrosionsbeständigen Edelstahl mit der Werkstoffkurzbezeichnung X20Cr13 (Werkstoffnummer: 1.4021) handeln. Dieser Edelstahl weist einen Kohlenstoffmassenanteil von 0,16 % bis 0,25 % und einen Chrommassenanteil von 12,0 % bis 14,0 % auf.

Alternativ kann es sich bei dem Edelstahl um einen martensitischen, korrosionsbeständigen Edelstahl mit der Werkstoffkurzbezeichnung X15Cr13 (Werkstoffnummer: 1.4024) handeln. Dieser Edelstahl weist einen Kohlenstoffmassenanteil von 0,12 % bis 0,17 % und einen Chrommassenanteil von 12,0 % bis 14,0 % auf.

Alternativ kann es sich bei dem Edelstahl um einen martensitischen, korrosionsbeständigen Edelstahl mit der Werkstoffkurzbezeichnung X30Cr13 (Werkstoffnummer: 1.4028) handeln. Dieser Edelstahl weist einen Kohlenstoffmassenanteil von 0,26 % bis 0,35 % sowie einen Chrommassenanteil von 12,0 % bis 14,0 % auf.

Alternativ kann es sich bei dem Edelstahl um einen martensitischen, korrosionsbeständigen Edelstahl mit der Werkstoffkurzbezeichnung X46Cr13 (Werkstoffnummer: 1.4034) handeln.

Dieser Edelstahl weist einen Kohlenstoffmassenanteil von 0,43 % bis 0,50 % sowie einen Chrommassenanteil von 12,5 % bis 14,5 % auf.

Alternativ kann es sich bei dem Edelstahl um einen martensitischen, korrosionsbeständigen Edelstahl mit der Werkstoffkurzbezeichnung X50CrMoV15 (Werkstoffnummer: 1.4116) handeln. Dieser Edelstahl weist einen Kohlenstoffmassenanteil von 0,45 % bis 0,55 %, einen Chrommassenanteil von 14,0 % bis 15,0 %, einen Molybdänmassenanteil von 0,50 % bis 0,80 % sowie einen Vanadiummassenanteil von 0,10 % bis 0,20 % auf.

Alternativ kann es sich bei dem Edelstahl um einen martensitischen, korrosionsbeständigen Edelstahl mit der Werkstoffkurzbezeichnung X17CrNi16-2 (Werkstoffnummer: 1.4057) handeln. Dieser Edelstahl weist einen Kohlenstoffmassenanteil von 0,12 % bis 0,22 %, einen Chrommassenanteil von 15,0 % bis 17,0 % sowie einen Nickelmassenanteil von 1,5 % bis 2,5 % auf.

Alternativ kann es sich bei dem Edelstahl um einen martensitischen, korrosionsbeständigen Edelstahl mit der Werkstoffkurzbezeichnung X39CrMo17-1 (Werkstoffnummer: 1.4122) handeln. Dieser Edelstahl weist einen Kohlenstoffmassenanteil von 0,33 % bis 0,45 %, einen Chrommassenanteil von 15,5 % bis 17,5 %, einen Molybdänmassenanteil von 0,8 % bis 1,3 % sowie einen Nickelmassenanteil ≤ 1,0 % auf.

Alternativ kann es sich bei dem Edelstahl um einen martensitischen, korrosionsbeständigen Edelstahl mit der Werkstoffkurzbezeichnung X14CrMoS17 (Werkstoffnummer: 1.4104) handeln. Dieser Edelstahl weist einen Kohlenstoffmassenanteil von 0,10 % bis 0,17 %, einen Chrommassenanteil von 15,5 % bis 17,5 %, einen Molybdänmassenanteil von 0,20 % bis 0,60 % sowie einen Schwefelmassenanteil von 0,15 % bis 0,35 % auf.

Alternativ kann es sich bei dem Edelstahl um einen martensitischen, korrosionsbeständigen Edelstahl mit der Werkstoffkurzbezeichnung X3CrNiMo13-4 (Werkstoffnummer: 1.4313) handeln. Dieser Edelstahl weist einen Kohlenstoffmassenanteil ≤ 0,05 %, einen Chrommassenanteil von 12,0 % bis 14,0 %, einen Molybdänmassenanteil von 0,3 % bis 0,7 % sowie einen Nickelmassenanteil von 3,5 % bis 4,5 % auf.

Alternativ kann es sich bei dem Edelstahl um einen martensitischen, korrosionsbeständigen Edelstahl mit der Werkstoffkurzbezeichnung X4CrNiMo16-5-1 (Werkstoffnummer: 1.4418) handeln. Dieser Edelstahl weist einen Kohlenstoffmassenanteil ≤ 0,06 %, einen Chrommassenanteil von 15,0 % bis 17,0 %, einen Molybdänmassenantei von 0,80 % bis 1,50 % sowie einen Nickelmassenanteil von 4,0 % bis 6,0 % auf.

Alternativ kann es sich bei dem Edelstahl um einen martensitischen Edelstahl mit der Werkstoffkurzbezeichnung X65Cr13 handeln. Dieser Edelstahl weist einen Kohlenstoffmassenanteil von 0,58 % bis 0,70 %, einen Chrommassenanteil von 12,5 % bis 14,5 %, einen Manganmassenanteil ≤ 1,00 %, einen Siliziummassenanteil ≤ 1,00 %, einen Phosphormassenanteil von 0,04 % sowie einen Schwefelmassenanteil von 0,015 % auf.

Alternativ kann es sich bei dem Edelstahl um einen martensitischen Edelstahl mit der Werkstoffkurzbezeichnung X30CrMoN15-1 (Werkstoffnummer: 1.4108) handeln. Dieser Edelstahl weist einen Kohlenstoffmassenanteil von 0,25 % bis 0,35 %, einen Chrommassenanteil von 14,0 % bis 16,0 %, einen Molybdänmassenanteil von 0,85 % bis 1,10 %, einen Nickelmassenanteil von 0,50 %, einen Manganmassenanteil von 1,00 %, einen Siliziummassenanteil von 1,00 % sowie einen Stickstoffmassenanteil von 0,03 % bis 0,50% auf.

Alternativ kann es sich bei dem Edelstahl um einen martensitischen Edelstahl mit der Werkstoffkurzbezeichnung X70CrMo15 (Werkstoffnummer: 1.4109) handeln. Dieser Edelstahl weist einen Kohlenstoffmassenanteil von 0,60 % bis 0,75 %, einen Chrommassenanteil von 14,0 % bis 16,0 %, einen Molybdänmassenanteil von 0,40 % bis 0,80 %, einen Manganmassenanteil ≤ 1,00 %, einen Siliziummassenanteil ≤ 0,70 %, einen Phosphormassenanteil von 0,04 % sowie einen Schwefelmassenanteil von 0,015 % auf.

Alternativ kann es sich bei dem Edelstahl um einen martensitischen Edelstahl mit der Werkstoffkurzbezeichnung X90CrMoV18 (Werkstoffnummer: 1.4112) handeln. Dieser Edelstahlweist einen Kohlenstoffmassenanteil von 0,90 %, einen Chrommassenanteil von 17 % bis 19 % sowie einen Molybdänmassenanteil von 0,90 % auf.

Alternativ kann es sich bei dem Edelstahl um einen martensitischen Edelstahl mit der Werkstoffkurzbezeichnung X38CrMoV15 (Werkstoffnummer: 1.4117) handeln. Dieser Edelstahl weist einen Kohlenstoffmassenanteil von 0,38 %, einen Chrommassenanteil von 14 % bis 15 % sowie einen Molybdänmassenanteil von 0,50 % auf.

Alternativ kann es sich bei dem Edelstahl um einen martensitischen Edelstahl mit der Werkstoffkurzbezeichnung X150CrMo17 (Werkstoffnummer: 1.4125) handeln. Dieser Edelstahl weist einen Kohlenstoffmassenanteil von 1,10 %, einen Chrommassenanteil von 17 % sowie einen Molybdänmassenanteil von 0,60 % auf.

Alternativ kann es sich bei dem Edelstahl um einen martensitischen Edelstahl mit der Werkstoffkurzbezeichnung X22CrMoNiS13-1 (Werkstoffnummer: 1.4121) handeln. Dieser Edelstahl weist einen Kohlenstoffmassenanteil von 0,20 % bis 0,25 %, einen Chrommassenanteil von 12,0 % bis 14,0 %, einen Molybdänmassenanteil von 1,00 % bis 1,50 %, einen Nickelmassenanteil von 0,80 % bis 1,20 %, einen Manganmassenanteil von 1,00 % bis 1,50 %, einen Siliziummassenanteil ≤ 1,00 %, einen Phosphormassenanteil von 0,045 % sowie einen Schwefelmassenanteil von 0,15 % bis 0,25 % auf.

Alternativ kann es sich bei dem Edelstahl um einen martensitischen Edelstahl mit der Werkstoffkurzbezeichnung X40CrMoVN16-2 (Werkstoffnummer: 1.4123) handeln. Dieser Edelstahlweist einen Kohlenstoffmassenanteil von 0,35 % bis 0,50 %, einen Chrommassenanteil von 14,0 % bis 16,0 %, einen Molybdänmassenanteil von 1,00 % bis 2,50 %, einen Nickelmassenanteil von 0,5 %, einen Manganmassenanteil ≤ 1,00 %, einen Siliziummassenanteil ≤ 1,00 %, einen Phosphormassenanteil von 0,04 % sowie einen Schwefelmassenanteil von 0,015 % auf.

Alternativ kann es sich bei dem Edelstahl um einen martensitischen Edelstahl mit der Werkstoffkurzbezeichnung X105CrMo17 (Werkstoffnummer: 1.4125) handeln. Dieser Edelstahl weist einen Kohlenstoffmassenanteil von 0,95 % bis 1,20 %, einen Chrommassenanteil von 16,0 % bis 18,0 %, einen Molybdänmassenanteil von 0,04 % bis 0,80 %, einen Manganmassenanteil von maximal 1,00 %, einen Silziummassenanteil von maximal 1,00 %, einen Phosphormassenanteil von maximal 0,040 % sowie einen Schwefelmassenanteil von maximal 0,015 % auf.

Alternativ kann es sich bei dem Edelstahl um einen ausscheidungshärtenden, korrosionsbeständigen Edelstahl mit der Werkstoffkurzbezeichnung X5CrNiCuNb16-4 (Werkstoffnummer: 1.4542) handeln. Dieser Edelstahl weist einen Kohlenstoffmassenanteil ≤ 0,07 %, einen Chrommassenanteil von 15,0 % bis 17,0 %, einen Molybdänmassenanteil ≤ 0,60 %, einen Nickelmassenanteil von 3,0 % bis 5,0 %, einen Kupfermassenanteil von 3,0 % bis 5,0 % sowie einen Niobmassenanteil von maximal 0,45 % auf.

Alternativ kann es sich bei dem Edelstahl um einen ausscheidungshärtenden, korrosionsbeständigen Edelstahl mit der Werkstoffkurzbezeichnung X7CrNiAl17-7 (Werkstoffnummer: 1.4568) handeln. Dieser Edelstahl weist einen Kohlenstoffmassenanteil ≤ 0,09 %, einen Chrommassenanteil von 16,0 % bis 18,0 %, einen Nickelmassenanteil von 6,5 % bis 7,8 % sowie einen Aluminiummassenanteil von 0,70 % bis 1,50 % auf.

Alternativ kann es sich bei dem Edelstahl um einen ausscheidungshärtenden, korrosionsbeständigen Edelstahl mit der Werkstoffkurzbezeichnung X5CrNiMoCuNb14-5 (Werkstoffnummer: 1.4594) handeln. Dieser Edelstahl weist einen Kohlenstoffmassenanteil ≤ 0,07 %, einen Chrommassenanteil von 13,0 % bis 15,0 %, einen Molybdänmassenanteil von 1,20 % bis 2,00 %, einen Nickelmassenanteil von 5,0 % bis 6,0 %, einen Kupfermassenanteil von 1,20 % bis 2,00 % sowie einen Niobmassenanteil von 0,15 % bis 0,60 % auf.

Alternativ kann es sich bei dem Edelstahl um einen ausscheidungshärtenden, korrosionsbeständigen Edelstahl mit der Werkstoffkurzbezeichnung X3CrNiTiMb12-9 (Werkstoffnummer: 1.4543) handeln. Dieser Edelstahl weist einen Kohlenstoffmassenanteil ≤ 0,03 %, einen Chrommassenanteil von 11,0 % bis 12,5 %, einen Molybdänmassenanteil ≤ 0,50%, einen Nickelmassenanteil von 3,00 % bis 5,00 %, einen Titanmassenanteil von ≤ 0,90 % bis 1,40 %, einem Kupfermassenanteil von 1,50 % bis 2,50 %, einen Niobmassenanteil von 0,10 % bis 0,50 %, einen Manganmassenanteil von 0,50 %, einen Siliziummassenanteil von 0,50 %, einen Phosphormassenanteil ≤ 0,02% sowie einen Schwefelmassenanteil ≤ 0,015 %.

Alternativ kann es sich bei dem Edelstahl um einen ferritischen, korrosionsbeständigen Edelstahl mit der Werkstoffkurzbezeichnung X2CrNi12 (Werkstoffnummer: 1.4003) handeln. Dieser Edelstahl weist einen Kohlenstoffmassenanteil ≤ 0,03 %, einen Chrommassenanteil von 10,5 % bis 12,5 %, einen Nickelmassenanteil von 0,3 % bis 1,00 % sowie einen Stickstoffanteil ≤ 0,03 % auf.

Alternativ kann es sich bei dem Edelstahl um einen ferritischen, korrosionsbeständigen Edelstahl mit der Werkstoffkurzbezeichnung X2CrNi12 (Werkstoffnummer: 1.4512) handeln. Dieser Edelstahl weist einen Kohlenstoffmassenanteil ≤ 0,03 %, einen Chrommassenanteil von 10,5 % bis 12,5 % sowie einen Titanmassenanteil von maximal 0,65 % auf.

Alternativ kann es sich bei dem Edelstahl um einen ferritischen, korrosionsbeständigen Edelstahl mit der Werkstoffkurzbezeichnung X6Cr17 (Werkstoffnummer: 1.4016) handeln. Dieser Edelstahl weist einen Kohlenstoffmassenanteil ≤ 0,08 % sowie einen Chrommassenanteil von 16,0 % bis 18,0 % auf.

Alternativ kann es sich bei dem Edelstahl um einen ferritischen, korrosionsbeständigen Edelstahl mit der Werkstoffkurzbezeichnung X3CrTi17 (Werkstoffnummer: 1.4510) handeln. Dieser Edelstahl weist einen Kohlenstoffmassenanteil ≤ 0,05 %, einen Chrommassenanteil von 16,0 % bis 18,0 % sowie einen Titanmassenanteil von maximal 0,80 % auf.

Alternativ kann es sich bei dem Edelstahl um einen ferritischen, korrosionsbeständigen Edelstahl mit der Werkstoffkurzbezeichnung X6CrMoS17 (Werkstoffnummer: 1.4105) handeln. Dieser Edelstahl weist einen Kohlenstoffmassenanteil ≤ 0,08 %, einen Chrommassenanteil von 16,0 % bis 18,0 %, einen Molybdänmassenanteil von 0,20 % bis 0,60 % sowie einen Schwefelmassenanteil von 0,15 % bis 0,35 % auf.

Alternativ kann es sich bei dem Edelstahl um einen ferritischen, korrosionsbeständigen Edelstahl mit der Werkstoffkurzbezeichnung X3CrNb17 (Werkstoffnummer: 1.4511) handeln. Dieser Edelstahl weist einen Kohlenstoffmassenanteil ≤ 0,05 %, einen Chrommassenanteil von 16,0 % bis 18,0 % sowie einen Niobmassenanteil von maximal 1,00 % auf.

Alternativ kann es sich bei dem Edelstahl um einen ferritischen, korrosionsbeständigen Edelstahl mit der Werkstoffkurzbezeichnung X2CrTiNb18 (Werkstoffnummer: 1.4509) handeln. Dieser Edelstahl weist einen Kohlenstoffmassenanteil ≤ 0,03 %, einen Chrommassenanteil von 17,5 % bis 18,5 %, einen Niobmassenanteil von maximal 1,00 % sowie einen Titanmassenanteil von 0,10 % bis 0,60 % auf.

Alternativ kann es sich bei dem Edelstahl um einen ferritischen, korrosionsbeständigen Edelstahl mit der Werkstoffkurzbezeichnung X6CrMo17-1 (Werkstoffnummer: 1.4113) handeln. Dieser Stahl weist einen Kohlenstoffmassenanteil ≤ 0,08 %, einen Chrommassenanteil von 16,0 % bis 18,0 % sowie einen Molybdänmassenanteil von 0,90 % bis 1,40 % auf.

Alternativ kann es sich bei dem Edelstahl um einen ferritischen, korrosionsbeständigen Edelstahl mit der Werkstoffkurzbezeichnung X2CrMoTi18-2 (Werkstoffnummer: 1.4521) handeln. Dieser Edelstahl weist einen Kohlenstoffmassenanteil ≤ 0,025 %, einen Chrommassenanteil von 17,0 % bis 20,0 %, einen Molybdänmassenanteil von 1,80 % bis 2,50 % sowie einen Titanmassenanteil von maximal 0,80 % auf.

Alternativ kann es sich bei dem Edelstahl um einen austenitisch-ferritischen, korrosionsbeständigen Edelstahl mit der Werkstoffkurzbezeichnung X2CrNi22-2 (Werkstoffnummer: 1.4062) handeln. Dieser Edelstahl weist einen Kohlenstoffmassenanteil ≤ 0,03 %, einen Chrommassenanteil von 21,5 % bis 24,0 %, einen Molybdänmassenanteil ≤ 0,45 %, einen Nickelmassenanteil von 1,00 % bis 2,90 % sowie einen Stickstoffmassenanteil von 0,16 % bis 0,28 % auf.

Alternativ kann es sich bei dem Edelstahl um einen austenitisch-ferritischen, korrosionsbeständigen Edelstahl mit der Werkstoffkurzbezeichnung X2CrMnNiN21-5-1 (Werkstoffnummer: 1.4162) handeln. Dieser Edelstahl weist einen Kohlenstoffmassenanteil ≤ 0,04 %, einen Chrommassenanteil von 21,0 % bis 22,0 %, einen Molybdänmassenanteil von 0,10 % bis 0,80 %, einen Nickelmassenanteil von 1,35 % bis 1,70 %, einen Manganmassenanteil von 4,0 % bis 6,0 %, einen Stickstoffmassenanteil von 0,20 % bis 0,25 % sowie einen Kupfermassenanteil von 0,10 % bis 0,80 % auf.

Alternativ kann es sich bei dem Edelstahl um einen austenitisch-ferritischen, korrosionsbeständigen Edelstahl mit der Werkstoffkurzbezeichnung X2CrNiN23-4 (Werkstoffnummer: 1.4362) handeln. Dieser Edelstahl weist einen Kohlenstoffmassenanteil ≤ 0,03 %, einen Chrommassenanteil von 22,0 % bis 24,0 %, einen Molybdänmassenanteil von 0,10 % bis 0,60 %, einen Nickelmassenanteil von 3,5 % bis 5,5 % sowie einen Kupfermassenanteil von 0,10 % bis 0,60 % auf.

Alternativ kann es sich bei dem Edelstahl um einen austenitisch-ferritischen, korrosionsbeständigen Edelstahl mit der Werkstoffkurzbezeichnung X2CrNiMoN22-5-3 (Werkstoffnummer: 1.4462) handeln. Dieser Edelstahl weist einen Kohlenstoffmassenanteil ≤ 0,03 %, einen Chrommassenanteil von 21,0 % bis 23,0 %, einen Molybdänmassenanteil von 2,5 % bis 3,5 %, einen Nickelmassenanteil von 4,5 % bis 6,5 % sowie einen Stickstoffmassenanteil von 0,10 % bis 0,22 % auf.

Alternativ kann es sich bei dem Edelstahl um einen austenitisch-ferritischen, korrosionsbeständigen Edelstahl mit der Werkstoffkurzbezeichnung X2CrNiMnMoCuN24-4-3-2 (Werkstoffnummer: 1.4662) handeln. Dieser Edelstahl weist einen Kohlenstoffmassenanteil ≤ 0,03 %, einen Chrommassenanteil von 23,0 % bis 25,0 %, einen Molybdänmassenanteil von 1,00 % bis 2,00 %, einen Nickelmassenanteil von 3,0 % bis 4,5 %, einen Manganmassenanteil von 2,5 % bis 4,0 % sowie einen Kupfermassenanteil von 0,10 % bis 0,80 % auf.

Alternativ kann es sich bei dem Edelstahl um einen austenitisch-ferritischen, korrosionsbeständigen Edelstahl mit der Werkstoffkurzbezeichnung X2CrNiMoN25-7-4 (Werkstoffnummer: 1.4410) handeln. Dieser Edelstahl weist einen Kohlenstoffmassenanteil ≤ 0,03 %, einen Chrommassenanteil von 24,0 % bis 26,0 %, einen Molybdänmassenanteil von 3,0 % bis 4,5 %, einen Nickelmassenanteil von 6,0 % bis 8,0 % sowie einen Stickstoffmassenanteil von 0,24 % bis 0,35 % auf.

Alternativ kann es sich bei dem Edelstahl um einen austenitisch-ferritischen, korrosionsbeständigen Edelstahl mit der Werkstoffkurzbezeichnung X2CrNiMoCuWN25-7-4 (Werkstoffnummer: 1.4501) handeln. Dieser Edelstahl weist einen Kohlenstoffmassenanteil ≤ 0,03 %, einen Chrommassenanteil von 24,0 % bis 26,0 %, einen Molybdänmassenanteil von 3,0 % bis 4,0 %, einen Nickelmassenanteil von 6,0 % bis 8,0 %, einen Kupfermassenanteil von 0,50 % bis 1,00 %, einen Wolframmassenanteil von 0,50 % bis 1,00 % sowie einen Stickstoffmassenanteil von 0,20 % bis 0,30 % auf.

Alternativ kann es sich bei dem Edelstahl um einen austenitischen, korrosionsbeständigen Edelstahl mit der Werkstoffkurzbezeichnung X2CrNiMo18-15-3 (Werkstoffnummer: 1.4441) handeln. Dieser Edelstahl weist einen Kohlenstoffmassenanteil von maximal 0,030 %, einen Chrommassenanteil von 17,0 % bis 19,0 %, einen Molybdänmassenanteil von 2,70 % bis 3,0 %, einen Nickelmassenanteil von 13,0 % bis 15,0 %, einen Manganmassenanteil von maximal 2,00 %, einen Kupfermassenanteil von maximal 0,50 %, einen Siliziummassenanteil von maximal 0,75 %, einen Phosphormassenanteil von maximal 0,025 %, einen Schwefelmassenanteil von maximal 0,003 % sowie einen Stickstoffmassenanteil von maximal 0,10 % auf.

Alternativ kann es sich bei dem Edelstahl um einen austenitischen, korrosionsbeständigen Edelstahl mit der Werkstoffkurzbezeichnung X5CrNi18-10 (Werkstoffnummer: 1.4301) handeln. Dieser Edelstahl weist einen Kohlenstoffmassenanteil ≤ 0,07 %, einen Chrommassenanteil von 17,5 % bis 19,5 %, einen Nickelmassenanteil von 8,0 % bis 10,5 % sowie einen Stickstoffmassenanteil ≤ 0,11 % auf.

Alternativ kann es sich bei dem Edelstahl um einen austenitischen, korrosionsbeständigen Edelstahl mit der Werkstoffkurzbezeichnung X4CrNi18-12 (Werkstoffnummer: 1.4303) handeln. Dieser Edelstahl weist einen Kohlenstoffmassenanteil ≤ 0,06 %, einen Chrommassenanteil von 17,0 % bis 19,0 %, einen Nickelmassenanteil von 11,0 % bis 13,0 % sowie einen Stickstoffmassenanteil ≤ 0,11 % auf.

Alternativ kann es sich bei dem Edelstahl um einen austenitischen, korrosionsbeständigen Edelstahl mit der Werkstoffkurzbezeichnung X8CrNiS18-9 (Werkstoffnummer: 1.4305) handeln. Dieser Edelstahl weist einen Kohlenstoffmassenanteil ≤ 0,10 %, einen Chrommassenanteil von 17,0 % bis 19,0 %, einen Nickelmassenanteil von 8,0 % bis 10,0 %, einen Schwefelmassenanteil von 0,15 % bis 0,35 % sowie einen Kupfermassenanteil ≤ 1,00 % auf.

Alternativ kann es sich bei dem Edelstahl um einen austenitischen, korrosionsbeständigen Edelstahl mit der Werkstoffkurzbezeichnung X2CrNi19-11 (Werkstoffnummer: 1.4306) handeln. Dieser Edelstahl weist einen Kohlenstoffmassenanteil ≤ 0,030 %, einen Chrommassenanteil von 18,0 % bis 20,0 %, einen Nickelmassenanteil von 10,0 % bis 12,0 % sowie einen Stickstoffmassenanteil ≤ 0,11 % auf.

Alternativ kann es sich bei dem Edelstahl um einen austenitischen, korrosionsbeständigen Edelstahl mit der Werkstoffkurzbezeichnung X2CrNi18-9 (Werkstoffnummer: 1.4307) handeln. Dieser Edelstahl weist einen Kohlenstoffmassenanteil ≤ 0,030 %, einen Chrommassenanteil von 17,5 % bis 19,5 %, einen Nickelmassenanteil von 8,0 % bis 10,5 % sowie einen Stickstoffmassenanteil ≤ 0,11 % auf.

Alternativ kann es sich bei dem Edelstahl um einen austenitischen, korrosionsbeständigen Edelstahl mit der Werkstoffkurzbezeichnung X2CrNi18-10 (Werkstoffnummer: 1.4311) handeln. Dieser Edelstahl weist einen Kohlenstoffmassenanteil ≤ 0,030 %, einen Chrommassenanteil von 17,5 % bis 19,5 %, einen Nickelmassenanteil von 8,5% bis 11,5 % sowie einen Stickstoffmassenanteil von 0,12 % bis 0,22 % auf.

Alternativ kann es sich bei dem Edelstahl um einen austenitischen, korrosionsbeständigen Edelstahl mit der Werkstoffkurzbezeichnung X6CrNiTi 18-10 (Werkstoffnummer: 1.4541) handeln. Dieser Edelstahl weist einen Kohlenstoffmassenanteil ≤ 0,08 %, einen Chrommassenanteil von 17,0 % bis 19,0 %, einen Nickelmassenanteil von 9,0 % bis 12,0 % sowie einen Titanmassenanteil von maximal 0,70 % auf.

Alternativ kann es sich bei dem Edelstahl um einen austenitischen, korrosionsbeständigen Edelstahl mit der Werkstoffkurzbezeichnung X6CrNiNb18-10 (Werkstoffnummer: 1.4550) handeln. Dieser Edelstahl weist einen Kohlenstoffmassenanteil ≤ 0,08 %, einen Chrommassenanteil von 17,0 % bis 19,0 %, einen Nickelmassenanteil von 9,0 % bis 12,0 % sowie einen Niobmassenanteil von maximal 1,00 % auf.

Alternativ kann es sich bei dem Edelstahl um einen austenitischen, korrosionsbeständigen Edelstahl mit der Werkstoffkurzbezeichnung X3CrNiCu18-9-4 (Werkstoffnummer: 1.4567) handeln. Dieser Edelstahl weist einen Kohlenstoffmassenanteil ≤ 0,04 %, einen Chrommassenanteil von 17,0 % bis 19,0 %, einen Nickelmassenanteil von 8,5 % bis 10,5 % sowie einen Kupfermassenanteil von 3,0 % bis 4,0 % auf.

Alternativ kann es sich bei dem Edelstahl um einen austenitischen, korrosionsbeständigen Edelstahl mit der Werkstoffkurzbezeichnung X10CrNi18-8 (Werkstoffnummer: 1.4310) handeln. Dieser Edelstahl weist einen Kohlenstoffmassenanteil von 0,05 % bis 0,15 %, einen Chrommassenanteil von 16,0 % bis 19,0 %, einen Molybdänmassenanteil ≤ 0,80 % sowie einen Nickelmassenanteil von 6,0 % bis 9,5 % auf.

Alternativ kann es sich bei dem Edelstahl um einen austenitischen, korrosionsbeständigen Edelstahl mit der Werkstoffkurzbezeichnung X5CrNiMo17-12-2 (Werkstoffnummer: 1.4401) handeln. Dieser Edelstahl weist einen Kohlenstoffmassenanteil ≤ 0,07 %, einen Chrommassenanteil von 16,5 % bis 18,5 %, einen Molybdänmassenanteil von 2,00 % bis 2,50 %, einen Nickelmassenanteil von 10,0 % bis 13,0 % sowie einen Stickstoffmassenanteil ≤ 0,10 % auf.

Alternativ kann es sich bei dem Edelstahl um einen austenitischen, korrosionsbeständigen Edelstahl mit der Werkstoffkurzbezeichnung X2CrNiMo17-12-2 (Werkstoffnummer: 1.4404) handeln. Dieser Edelstahl weist einen Kohlenstoffmassenanteil ≤ 0,030 %, einen Chrommassenanteil von 16,5 % bis 18,5 %, einen Molybdänmassenanteil von 2,00 % bis 2,50 %, einen Nickelmassenanteil von 10,0 % bis 13,0 % sowie einen Stickstoffmassenanteil ≤ 0,10 % auf.

Alternativ kann es sich bei dem Edelstahl um einen austenitischen, korrosionsbeständigen Edelstahl mit der Werkstoffkurzbezeichnung X6CrNiMoTi17-12-2 (Werkstoffnummer: 1.4571) handeln. Dieser Edelstahl weist einen Kohlenstoffmassenanteil ≤ 0,08 %, einen Chrommassenanteil von 16,5 % bis 18,5 %, einen Molybdänmassenanteil von 2,00 % bis 2,50 %, einen Nickelmassenanteil von 10,5 % bis 13,5 % sowie einen Titanmassenanteil von maximal 0,70 % auf.

Alternativ kann es sich bei dem Edelstahl um einen austenitischen, korrosionsbeständigen Edelstahl mit der Werkstoffkurzbezeichnung X2CrNiMoN17-13-3 (Werkstoffnummer: 1.4429) handeln. Dieser Edelstahl weist einen Kohlenstoffmassenanteil ≤ 0,030 %, einen Chrommassenanteil von 16,5 % bis 18,5 %, einen Molybdänmassenanteil von 2,5 % bis 3,0 %, einen Nickelmassenanteil von 11,0 % bis 14,0 % sowie einen Stickstoffmassenanteil von 0,12 % bis 0,22 % auf.

Alternativ kann es sich bei dem Edelstahl um einen austenitischen, korrosionsbeständigen Edelstahl mit der Werkstoffkurzbezeichnung X2CrNiMo18-14-3 (Werkstoffnummer: 1.4435) handeln. Dieser Edelstahl weist einen Kohlenstoffmassenanteil ≤ 0,030 %, einen Chrommassenanteil von 17,0 % bis 19,0 %, einen Molybdänmassenanteil von 2,5 % bis 3,0 %, einen Nickelmassenanteil von 12,5 % bis 15,0 % sowie einen Stickstoffmassenanteil ≤ 0,10 % auf.

Alternativ kann es sich bei dem Edelstahl um einen austenitischen, korrosionsbeständigen Edelstahl mit der Werkstoffkurzbezeichnung X3CrNiMo17-13-3 (Werkstoffnummer: 1.4436) handeln. Dieser Edelstahl weist einen Kohlenstoffmassenanteil ≤ 0,05 %, einen Chrommassenanteil von 16,5 % bis 18,5 %, einen Molybdänmassenanteil von 2,5 % bis 3,0 %, einen Nickelmassenanteil von 10,5 % bis 13,0 % sowie einen Stickstoffmassenanteil ≤ 0,10 % auf.

Alternativ kann es sich bei dem Edelstahl um einen austenitischen, korrosionsbeständigen Edelstahl mit der Werkstoffkurzbezeichnung X2CrNiMoN17-13-5 (Werkstoffnummer: 1.4439) handeln. Dieser Edelstahl weist einen Kohlenstoffmassenanteil ≤ 0,030 %, einen Chrommassenanteil von 16,5 % bis 18,5 %, einen Molybdänmassenanteil von 4,0 % bis 5,0 %, einen Nickelmassenanteil von 12,5 % bis 14,5 % sowie einen Stickstoffmassenanteil von 0,12 % bis 0,22 % auf.

Alternativ kann es sich bei dem Edelstahl um einen austenitischen, korrosionsbeständigen Edelstahlmit der Werkstoffkurzbezeichnung X1NiCrMoCu25-20-5 (Werkstoffnummer: 1.4539) handeln. Dieser Edelstahl weist einen Kohlenstoffmassenanteil ≤ 0,020 %, einen Chrommassenanteil von 19,0 % bis 21,0 %, einen Molybdänmassenanteil von 4,0 % bis 5,0 %, einen Nickelmassenanteil von 24,0 % bis 26,0 %, einen Kupfermassenanteil von 1,20 % bis 2,00 % sowie einen Stickstoffmassenanteil ≤ 0,15 % auf.

Alternativ kann es sich bei dem Edelstahl um einen austenitischen, korrosionsbeständigen Edelstahl mit der Werkstoffkurzbezeichnung X2CrNiMnMoNbN25-18-5-4 (Werkstoffnummer: 1.4565) handeln. Dieser Edelstahl weist einen Kohlenstoffmassenanteil ≤ 0,030 %, einen Chrommassenanteil von 24,0 % bis 26,0 %, einen Molybdänmassenanteil von 4,0 % bis 5,0 %, einen Nickelmassenanteil von 16,0 % bis 19,0 %, einen Manganmassenanteil von 5,0 % bis 7,0 %, einen Stickstoffmassenanteil von 0,30 % bis 0,60 % sowie einen Niobmassenanteil ≤ 0,15 % auf.

Alternativ kann es sich bei dem Edelstahl um einen austenitischen, korrosionsbeständigen Edelstahl mit der Werkstoffkurzbezeichnung X1NiCrMoCuN25-20-7 (Werkstoffnummer: 1.4529) handeln. Dieser Edelstahl weist einen Kohlenstoffmassenanteil ≤ 0,020 %, einen Chrommassenanteil von 19,0 % bis 21,0 %, einen Molybdänmassenanteil von 6,0 % bis 7,0 %, einen Nickelmassenanteil von 24,0 % bis 26,0 %, einen Kupfermassenanteil von 0,50 % bis 1,50 % sowie einen Stickstoffmassenanteil von 0,15 % bis 0,25 % auf.

Alternativ kann es sich bei dem Edelstahl um einen austenitischen, korrosionsbeständigen Edelstahl mit der Werkstoffkurzbezeichnung X1CrNiMoCuN20-18-7 (Werkstoffnummer: 1.4547) handeln. Dieser Edelstahl weist einen Kohlenstoffmassenanteil ≤ 0,020 %, einen Chrommassenanteil von 19,5 % bis 20,5 %, einen Molybdänmassenanteil von 6,0 % bis 7,0 %, einen Nickelmassenanteil von 17,5 % bis 18,5 %, einen Kupfermassenanteil von 0,50 % bis 1,00 % sowie einen Stickstoffmassenanteil von 0,18 % bis 0,25 % auf.

Alternativ kann es sich bei dem Edelstahl um einen austenitischen, korrosionsbeständigen Edelstahl mit der Werkstoffkurzbezeichnung X1CrNiMoCuN24-22-8 (Werkstoffnummer: 1.4652) handeln. Dieser Edelstahl weist einen Kohlenstoffmassenanteil ≤ 0,020 %, einen Chrommassenanteil von 23,0 % bis 25,0 %, einen Molybdänmassenanteil von 7,0 % bis 8,0 %, einen Nickelmassenanteil von 21,0 % bis 23,0 %, einen Manganmassenanteil von 2,0 % bis 4,0 % sowie einen Stickstoffmassenanteil von 0,45 % bis 0,55 % auf.

Bei dem medizintechnischen Produkt handelt es sich um ein chirurgisches Instrument. Bei dem Instrument kann es sich um ein wiederverwendbares Instrument oder um ein Einweginstrument ("single use instrument") handeln.

Ferner kann es sich bei dem Instrument um ein minimalinvasives Instrument, d.h. um ein in der minimalinvasiven Chirurgie einsetzbares Instrument, handeln.

Das chirurgische Instrument kann insbesondere aus der Gruppe bestehend aus spreizendes Instrument, fassendes Instrument, klemmendes Instrument, schneidendes Instrument, Nahtgerät, Endoskop und kombiniertes Instrument ausgewählt sein.

Bei dem spreizenden Instrument kann es sich beispielsweise um einen Wundhaken, einen Retraktor, einen Wundspreizer, einen Brustbeinspreizer, einen Wundsperrer, ein Spekulum oder eine Trokarhülse handeln.

Bei dem fassenden Instrument kann es sich beispielsweise um eine Pinzette, eine Klemme, einen Nadelhalter oder eine Fasszange handeln.

Bei dem klemmenden Instrument kann es sich beispielsweise um eine weiche Klemme, insbesondere zum temporären Abklemmen von Darm und feinen Gefäßen, oder um eine Präparierklemme handeln.

Bei dem schneidenden Instrument kann es sich beispielsweise um ein Skalpell, ein Messer, eine Schere, eine Branchenzange, eine Knochensplitterzange, eine Ringzange, ein Elektrotom, ein Konchotom, einen Kauter oder ein Ultraschallmesser handeln.

Bei dem Nahtgerät kann es sich insbesondere um ein Klammernahtgerät (Stapler) oder um einen Klammerentferner handeln.

Bei dem kombinierten Instrument kann es sich um einen Endostapler oder ein Klammernahtgerät, welches beispielsweise ein Hohlorgan verklammert und zugleich präzise schneidet, handeln. Weiterhin kann es sich bei dem kombinierten Instrument um einen kombinierten Nadelhalter, der als Universalnahtgerät sowohl fassen als auch schneiden kann, handeln.

Ferner kann es sich bei dem chirurgischen Instrument um einen Hammer handeln.

Ferner kann es sich bei dem chirurgischen Instrument um einen Meißel, insbesondere Flach- oder Hohlmeißel wie Knochenhohlmeißel, oder um eine Kürette, insbesondere Knochenkürette, handeln.

Ferner kann es sich bei dem chirurgischen Instrument um eine Sonde handeln.

Ferner kann es sich bei dem chirurgischen Instrument um eine Knochenstanze handeln.

Ferner kann es sich bei dem chirurgischen Instrument um einen Hebel oder Elevator oder ein Raspatorium handeln.

Gemäß einem zweiten Aspekt betrifft die Erfindung ein chirurgisches Instrument oder Bauteil davon, aufweisend oder bestehend aus einem chromhaltigen Edelstahl, wobei das chirurgische Instrument oder Bauteil davon hergestellt oder herstellbar ist nach einem Verfahren gemäß erstem Erfindungsaspekt und folgendes Merkmal aufweist:
- eine Passivschicht, insbesondere aus Chromoxid, mit einer Dicke von 1 nm bis 10 nm, insbesondere 3 nm bis 10 nm, bevorzugt 5 nm bis 10 nm, welche die Oberfläche des medizintechnischen Produkts wenigstens abschnittsweise, insbesondere nur abschnittsweise oder vollständig, beschichtet.

Die vorgenannten Lochkorrosionspotentiale sowie Kontaktwinkel sind im Hinblick auf die Korrosionsbeständigkeit des medizintechnischen Produkts besonders vorteilhaft.

Unter dem Ausdruck "Lochkorrosionspotential" soll im Sinne der vorliegenden Erfindung das elektrochemische Potential verstanden werden, das mit einer elektrochemischen Zelle unter Verwendung einer Drei-Elektroden-Anordnung bestimmt werden kann. Das Lochkorrosionspotential ist durch einen schnellen Stromanstieg charakterisiert und beschreibt dabei den Zusammenbruch der Passivschicht unter einsetzender Lochkorrosion. Eine Erhöhung des Lochkorrosionspotentials bewirkt eine Verbesserung der Korrosionsbeständigkeit durch eine Reduzierung der Lochkorrosionsanfälligkeit.

Die Messung des Lochkorrosionspotentials kann gemäß ASTM G5-13-1 oder DIN EN ISO 10993-15 erfolgen.

Unter dem Ausdruck "Kontaktwinkel" soll im Sinne der vorliegenden Erfindung der Winkel verstanden werden, welchen ein Flüssigkeitstropfen auf der Oberfläche des medizintechnischen Produkts zu dessen Oberfläche bildet. Ein verringerter Kontaktwinkel geht mit einem verringerten Kontakt des Flüssigkeitstropfens an der Oberfläche des medizintechnischen Produkts einher. Eine Verringerung des Kontaktwinkels bewirkt mit besonderem Vorteil eine Verbesserung der Korrosionsbeständigkeit sowie Reinigbarkeit des medizintechnischen Produkts.

Die Messung des Kontaktwinkels kann gemäß ASTM D 7334-08 erfolgen. Alternativ kann die Messung des Kontaktwinkels mittels eines Kontaktwinkelmessgeräts der Firma dataPhysics (Contact Angle System OCA 15 Plus) und unter Verwendung einer 0,9 %igen NatriumchloridLösung (B.Braun) durchgeführt werden, wobei das Tropfenvolumen 1 µl beträgt. Zur Messung des Kontaktwinkels können die Proben in diesem Fall in einem regulären Fertigungsprozess ausgewaschen und vor der Messung in vollentsalztem Wasser im Ultraschallbad für 5 Minuten gereinigt werden, wobei die Proben direkt vor der Messung mit vollentsalztem Wasser abgewaschen und mit ölfreier Druckluft abgeblasen werden.

Bei dem medizintechnischen Produkt handelt es sich um ein chirurgisches Instrument oder ein Bauteil eines chirurgischen Instruments.

Bezüglich weiterer Merkmale und Vorteile des medizintechnischen Produkts wird zur Vermeidung von Wiederholungen vollständig auf die im Rahmen des ersten Erfindungsaspekts gemachten Ausführungen Bezug genommen. Die dort in Bezug auf das Verfahren sowie das medizintechnische Produkt beschriebenen Merkmale und Vorteile gelten sinngemäß auch für das medizintechnische Produkt gemäß zweitem Erfindungsaspekt.

Weitere Merkmale und Vorteile der Erfindung ergeben sich aus den Ansprüchen sowie aus der nachfolgenden Beschreibung bevorzugter Ausführungsformen anhand von Beispielen.

Die nachfolgend beschriebenen Ausführungsformen dienen der weiteren Erläuterung der Erfindung, ohne diese hierauf zu beschränken.

### BEISPIELTEIL

1. Oberflächenbehandlung eines chirurgischen Instruments bzw. repräsentativer Probekörper nach einem erfindungsgemäßen Verfahren

Die verwendeten Probekörper als auch chirurgischen Instrumente wurden aus identischem martensitischen nichtrostenden Stahl (X20Cr13) und nach identischen Fertigungsschritten sowie -parametern hergestellt.

REM/EDX-Analysen (Fremdmaterial und Materialdopplungen) wurden an den Instrumenten und Probeplättchen durchgeführt.

Potentiodynamische Prüfungen (Lochkorrosionspotential) wurden ebenfalls an den Instrumenten und Probeplättchen durchgeführt.

Kontaktwinkelmessungen (Kontaktwinkel) wurden an Probeplättchen (plane Fläche ohne Abschattungen) durchgeführt.

Glanzmessung (Glanzgrad) wurden an Probeplättchen (plane Fläche ohne Abschattungen) durchgeführt.

3D-Laserkonfokalmikroskopie (Rautiefe) wurden an Probeplättchen (plane Fläche ohne Abschattungen) durchgeführt.

Vor der Oberflächenbehandlung wurden chirurgische Instrumente, Korrosionsprobekörper und Probenplättchen nach der aktuellen Herstellungskette chirurgischer Instrumente in Form gebracht und wärmebehandelt.

Zur anschließenden Oberflächenbehandlung wurden ein chirurgisches Instrument (Klemme BH110R), ein Korrosionsprobekörper und Probeplättchen während eines Zeitraumes von vier Stunden mittels Gleitschleifen in saurer Lösung behandelt und anschließend durch Gleitschleifen in wässriger Lösung über einen Zeitraum von einer Stunde aufgehellt.

Danach wurden die chirurgischen Instrumente, Korrosionsprobekörper und Probenplättchen elektrochemisch geätzt. Hierfür wurden die Teile in eine auf 40°C temperierte saure wässrige Elektrolytlösung mit einem Mineralsäureanteil von 11 Gew-% Phosphorsäure und 61 Gew-% Schwefelsäure getaucht und für 10 Minuten eine Gleichspannung angelegt, damit an der Anode eine Spannung von 1,5 V entsteht. Es stellte sich dabei eine Stromdichte von 2,0 A/dm² ein.

Abschließend wurden die chirurgischen Instrumente, Korrosionsprobekörper und Probenplättchen passiviert. Hierfür wurden die Teile für 10 Minuten in eine auf 60°C temperierte 10 Gew-% Zitronensäurelösung getaucht. Hiernach wurden die Teile dekapiert und in Ethanol gereinigt.

Nach der Herstellung wurde die Ausbildung der Oberfläche von Instrumenten und Probeplättchen über die Rasterelektronenmikroskopie samt energiedispersiver Röntgenspektroskopie-Einheit untersucht. Die REM-Untersuchungen zeigten nahezu statistisch über die Oberfläche verteilte Ätzgrübchen mit leichter Lokalisierung an den Korngrenzen. Selbige lagen im Größenordnungsbereich von etwa 5 µm. Die chemische Zusammensetzung war homogen und im Vergleich zum Ausgangsmaterial um etwa 0,1 Gew-% an Chrom ärmer. Selbiges lag an den aus der Oberfläche gelösten Chromcarbiden.

Weiterhin wurde die topografische Ausprägung der Oberfläche an den Instrumenten und Probeplättchen mittels 3D-Laserkonfokalmikroskopie und durch metallografische Querschliffe bewertet. Durch die 3D-Laserkonfokalmessungen konnte eine gemittelte Rautiefe von 0,5 µm bestimmt werden. Selbiges war auf die Tiefe der Ätzgrübchen zurückzuführen, die nach den metallografischen Untersuchungen im Bereich von 1-3 µm lagen.

Die Änderung des Reflexionsverhaltens wurde mit einer Glanzmessung an den Prüfplättchen untersucht. Es zeigte sich ein deutlich reduzierter Glanz mit Werten der Glossunits (20°) von 3,7 und Glossunits (60°) von 21,6. Das Reflexionsverhalten konnte somit als stark matt bestimmt werden.

Eine Analyse der Benetzung durch Flüssigkeiten geschah durch Kontaktwinkelmessung an den Prüfplättchen. Hierbei wurde ein Mittelwert des Kontaktwinkels von 116,3° bestimmt.

Abschließend wurde das elektrochemische / korrosive Verhalten der ausgebildeten Oberfläche durch potentiodynamische Polarisationsmessungen an Korrosionsprobekörpern untersucht und das Lochkorrosionspotential ermittelt. Zum Vergleich, ob die Messwerte, die an Probekörpern gemessen wurden, sich auf das Instrument beziehen lassen, wurde das Lochkorrosionspotential an einem Labor Instrumente gemessen. Hierbei wurden die Ergebnisse der Probekörper bestätigt. Hierbei konnte ein Lochkorrosionspotential von 475 mV aufgenommen werden.

2. Oberflächenbehandlung eines chirurgischen Instruments nach einem gattungsgemäßen Verfahren

Ein chirurgisches Instrument (Klemme BH110R), Korrosionsprobekörper und Probeplättchen wurden zunächst während eines Zeitraumes von vier Stunden mittels Gleitschleifen behandelt. Danach ließ man das chirurgische Instrument und die Probekörper während eines Zeitraumes von einer Stunde aufhellen.

Danach wurden das chirurgische Instrument und die Probekörper mittels Strahlen behandelt. Hierzu wurden Glasperlen mit einem mittleren Durchmesser von 40 µm bis 70 µm verwendet. Das Strahlen wurde in einer Injektorstrahlanlage unter einem Druck von 4 bar durchgeführt.

Anschließend wurden das chirurgische Instrument und die Probekörper einer Passivierung unterworfen. Hierzu wurde eine 10 %ige Zitronensäurelösung verwendet. Das Passivieren erfolgte während eines Zeitraumes von 10 Minuten bei einer Temperatur von 55 °C.

Nach Abschluss der Oberflächenbehandlung des chirurgischen Instruments und der Probekörper waren viele Materialdopplungen bzw. Materialüberlappungen feststellbar. Es konnte zudem ein Fremdmaterialübertrag von 1,4 % nachgewiesen werden. Die Rautiefe lag in Bereichen von 0,151 µm. Außerdem wiesen die Probeplättchen einen Kontaktwinkel von 66,0 ° auf. Der Glanzgrad konnte auf Glossunits (20°) von 41,9 und Glossunits (60°) von 159,8 bestimmt und somit als leicht matt ausgemacht werden. Das Lochkorrosionspotential der Korrosionsprobekörper betrug 386 mV.

### 3. Fazit

Die oben beschriebene Gegenüberstellung eines erfindungsgemäßen Verfahrens und eines gattungsgemäßen Verfahrens zeigt, dass das erfindungsgemäße Verfahren zu korrosionsbeständigeren Produkten mit einer sehr geringen Reflexion (Glanzgrad) führt.

## Patentansprüche

1. Verfahren zum Herstellen eines chirurgischen Instruments oder eines Bauteils eines chirurgischen Instruments, wobei das chirurgische Instrument oder Bauteil eines chirurgischen Instruments chromhaltigen Edelstahl aufweist oder aus chromhaltigem Edelstahl besteht, wobei das Verfahren folgenden Schritt aufweist:
a) elektrochemisches Ätzen des chirurgischen Instruments oder des Bauteils eines chirurgischen Instruments, **dadurch gekennzeichnet, dass** der Schritt a) bei einer an der Anode anliegenden Spannung von 1,4 V bis 1,7 V und/oder bei einer Stromdichte von 1,6 A/dm² bis 2,2 A/dm² durchgeführt wird.

2. Verfahren nach Anspruch 1, **dadurch gekennzeichnet, dass** vor dem Durchführen von Schritt a) ein Schleifen, vorzugsweise Gleit- und/oder Bandschleifen, der Oberfläche des chirurgischen Instruments oder des Bauteils eines chirurgischen Instruments durchgeführt wird.

3. Verfahren nach Anspruch 1 oder 2, **dadurch gekennzeichnet, dass** die Oberfläche des chirurgischen Instruments oder des Bauteils eines chirurgischen Instruments nicht mit einem Strahlmittel behandelt und/oder nicht elektropoliert wird.

4. Verfahren nach einem der vorhergehenden Ansprüche, **dadurch gekennzeichnet, dass** der Schritt a) mehrmals, insbesondere zwei-, drei- oder viermal, durchgeführt wird.

5. Verfahren nach einem der vorhergehenden Ansprüche, **dadurch gekennzeichnet, dass** zum Durchführen von Schritt a) eine saure, wässrige Elektrolytlösung, aufweisend eine Mineralsäure oder ein Mineralsäuregemisch, verwendet wird.

6. Verfahren nach Anspruch 5, **dadurch gekennzeichnet, dass** die Mineralsäure ausgewählt wird aus der Gruppe bestehend aus Phosphorsäure, Schwefelsäure und ein Gemisch davon.

7. Verfahren nach einem der vorhergehenden Ansprüche, **dadurch gekennzeichnet, dass** der Schritt a) während eines Zeitraumes von 6 min bis 14 min, insbesondere 8 min bis 12 min, vorzugsweise von 10 min, durchgeführt wird.

8. Verfahren nach einem der vorhergehenden Ansprüche, **dadurch gekennzeichnet, dass** der Schritt a) bei einer an der Anode anliegenden Spannung von 1,45 V bis 1,65 V durchgeführt wird.

9. Verfahren nach einem der vorhergehenden Ansprüche, **dadurch gekennzeichnet, dass** der Schritt a) bei einer Stromdichte von 1,8 A/dm² bis 2,0 A/dm² durchgeführt wird.

10. Verfahren nach einem der vorhergehenden Ansprüche, **dadurch gekennzeichnet, dass** der Schritt a) bei einer Temperatur von 20 °C bis 90 °C, insbesondere 50 °C bis 80 °C, vorzugweise 70 °C bis 80 °C, durchgeführt wird.

11. Verfahren nach einem der vorhergehenden Ansprüche, **dadurch gekennzeichnet, dass** die Oberfläche des chirurgischen Instruments oder des Bauteils eines chirurgischen Instruments, insbesondere nach Durchführen von Schritt a), nicht mit einer Passiviersäure oder einer passiviersäurehaltigen Lösung behandelt wird.

12. Verfahren nach einem der vorhergehenden Ansprüche, **dadurch gekennzeichnet, dass** nach dem Durchführen von Schritt a) ein Schritt b) Verpacken des chirurgischen Instruments oder des Bauteils eines chirurgischen Instruments und zwischen dem Schritt a) und dem Schritt b) ein Schritt ab) Sterilisieren des chirurgischen Instruments oder des Bauteils eines chirurgischen Instruments oder nach dem Durchführen von Schritt b) ein Schritt c) Sterilisieren des chirurgischen Instruments oder des Bauteils eines chirurgischen Instruments durchgeführt wird.

13. Verfahren nach einem der vorhergehenden Ansprüche, **dadurch gekennzeichnet, dass** das chirurgische Instrument oder das Bauteil eines chirurgischen Instruments aus einem chromhaltigen, korrosionsbeständigen Edelstahl, insbesondere martensitischen, korrosionsbeständigen Edelstahl, besteht.

14. Chirurgisches Instrument oder Bauteil eines chirurgischen Instruments, aufweisend oder bestehend aus chromhaltigem Edelstahl, wobei das chirurgische Instrument oder das Bauteil eines chirurgischen Instruments herstellbar ist nach einem Verfahren nach einem der vorhergehenden Ansprüche und folgendes Merkmal aufweist:
- eine Passivschicht mit einer Dicke von 1 nm bis 10 nm, welche die Oberfläche des chirurgischen Instruments oder des Bauteils eines chirurgischen Instruments wenigstens abschnittsweise beschichtet.

## Claims

1. Method of producing a surgical instrument or a component of a surgical instrument, wherein the surgical instrument or component of a surgical instrument includes chromium-containing stainless steel or consists of chromium-containing stainless steel, wherein the method includes the following step:
a) electrochemically etching the surgical instrument or the component of a surgical instrument, **characterized in that** step a) is conducted at a voltage applied to the anode of 1.4 V to 1.7 V and/or at a current density of 1.6 A/dm² to 2.2 A/dm².

2. Method according to Claim 1, **characterized in that** the performance of step a) is preceded by a finishing operation, preferably slide finishing and/or belt finishing, of the surface of the surgical instrument or of the component of a surgical instrument.

3. Method according to Claim 1 or 2, **characterized in that** the surface of the surgical instrument or of the component of a surgical instrument is not treated with an abrasive and/or is not electropolished.

4. Method according to any of the preceding claims, **characterized in that** step a) is performed repeatedly, especially twice, three times or four times.

5. Method according to any of the preceding claims, **characterized in that** step a) is conducted using an acidic aqueous electrolyte solution including a mineral acid or a mineral acid mixture.

6. Method according to Claim 5, **characterized in that** the mineral acid is selected from the group consisting of phosphoric acid, sulfuric acid and a mixture thereof.

7. Method according to any of the preceding claims, **characterized in that** step a) is conducted over a period of 6 min to 14 min, especially 8 min to 12 min, preferably of 10 min.

8. Method according to any of the preceding claims, **characterized in that** step a) is performed at a voltage applied to the anode of 1.45 V to 1.65 V.

9. Method according to any of the preceding claims, **characterized in that** step a) is performed at a current density of 1.8 A/dm² to 2.0 A/dm².

10. Method according to any of the preceding claims, **characterized in that** step a) is conducted at a temperature of 20°C to 90°C, especially 50°C to 80°C, preferably 70°C to 80°C.

11. Method according to any of the preceding claims, **characterized in that** the surface of the surgical instrument or of the component of a surgical instrument, especially after performance of step a), is not treated with a passivating acid or a passivating acid-containing solution.

12. Method according to any of the preceding claims, **characterized in that** a step b) of packaging the surgical instrument or the component of a surgical instrument is performed after step a) has been performed and a step ab) of sterilizing the surgical instrument or the component of a surgical instrument is performed between step a) and step b) or a step c) of sterilizing the surgical instrument or the component of a surgical instrument is performed after step b) has been performed.

13. Method according to any of the preceding claims, **characterized in that** the surgical instrument or the component of a surgical instrument consists of a chromium-containing, corrosion-resistant stainless steel, especially martensitic, corrosion-resistant stainless steel.

14. Surgical instrument or component of a surgical instrument, including or consisting of chromium-containing stainless steel, wherein the surgical instrument or the component of a surgical instrument is producible by a method according to any of the preceding claims, and includes the following feature:
- a passivation layer having a thickness of 1 nm to 10 nm that coats the surface of the surgical instrument or of the component of a surgical instrument at least in sections.

## Revendications

1. Procédé pour la fabrication d'un instrument chirurgical ou d'un composant d'un instrument chirurgical, dans lequel l'instrument chirurgical ou le composant d'instrument chirurgical comprend de l'acier inoxydable contenant du chrome ou est constitué d'acier inoxydable contenant du chrome, le procédé comprenant l'étape suivante :
a) attaque électrochimique de l'instrument chirurgical ou du composant d'un instrument chirurgical, **caractérisé en ce que** l'étape a) est effectuée sous une tension appliquée à l'anode de 1,4 V à 1,7 V et/ou à une densité de courant de 1,6 A/dm² à 2,2 A/dm².

2. Procédé selon la revendication 1, **caractérisé en ce que**, avant l'exécution de l'étape a), est effectué un ponçage, de préférence ponçage par glissement et/ou à la bande abrasive, de la surface de l'instrument chirurgical ou du composant d'un instrument chirurgical.

3. Procédé selon la revendication 1 ou 2, **caractérisé en ce que** la surface de l'instrument chirurgical ou du composant d'un instrument chirurgical n'est pas traitée par un agent de sablage et/ou n'est pas électropolie.

4. Procédé selon l'une quelconque des revendications précédentes, **caractérisé en ce que** l'étape a) est effectuée plusieurs fois, en particulier deux, trois ou quatre fois.

5. Procédé selon l'une quelconque des revendications précédentes, **caractérisé en ce que** pour l'exécution de l'étape a) est utilisée une solution électrolytique aqueuse, acide, comportant un acide minéral ou un mélange d'acides minéraux.

6. Procédé selon la revendication 5, **caractérisé en ce que** l'acide minéral est choisi dans le groupe constitué par l'acide phosphorique, l'acide sulfurique et un mélange de ceux-ci.

7. Procédé selon l'une quelconque des revendications précédentes, **caractérisé en ce que** l'étape a) est effectuée pendant une durée de 6 min à 14 min, en particulier 8 min à 12 min, de préférence de 10 min.

8. Procédé selon l'une quelconque des revendications précédentes, **caractérisé en ce que** l'étape a) est effectuée sous une tension appliquée à l'anode de 1,45 V à 1,65 V.

9. Procédé selon l'une quelconque des revendications précédentes, **caractérisé en ce que** l'étape a) est effectuée à une densité de courant de 1,8 A/dm² à 2,0 A/dm ².

10. Procédé selon l'une quelconque des revendications précédentes, **caractérisé en ce que** l'étape a) est effectuée à une température de 20 °C à 90 °C, en particulier 50 °C à 80 °C, de préférence 70 °C à 80 °C.

11. Procédé selon l'une quelconque des revendications précédentes, **caractérisé en ce que** la surface de l'instrument chirurgical ou du composant d'un instrument chirurgical, en particulier après exécution de l'étape a), n'est pas traitée par un acide de passivation ou une solution contenant un acide de passivation.

12. Procédé selon l'une quelconque des revendications précédentes, **caractérisé en ce que**, après l'exécution de l'étape a), est effectuée une étape b) d'emballage de l'instrument chirurgical ou du composant d'un instrument chirurgical et, entre l'étape a) et l'étape b), est effectuée une étape ab) de stérilisation de l'instrument chirurgical ou du composant d'un instrument chirurgical, ou, après l'exécution de l'étape b), est effectuée une étape c) de stérilisation de l'instrument chirurgical ou du composant d'un instrument chirurgical.

13. Procédé selon l'une quelconque des revendications précédentes, **caractérisé en ce que** l'instrument chirurgical ou le composant d'un instrument chirurgical est constitué d'un acier inoxydable résistant à la corrosion, contenant du chrome, en particulier d'acier inoxydable résistant à la corrosion, martensitique.

14. Instrument chirurgical ou composant d'instrument chirurgical comportant de l'acier inoxydable contenant du chrome ou constitué d'un tel acier, l'instrument chirurgical ou le composant d'instrument chirurgical pouvant être fabriqué conformément à un procédé selon l'une quelconque des revendications précédentes et présentant la caractéristique suivante :
- une couche de passivation ayant une épaisseur de 1 nm à 10 nm, qui recouvre au moins par parties la surface de l'instrument chirurgical ou du composant d'un instrument chirurgical.
